# EUROPÄISCHE PATENTSCHRIFT

(11) **EP 2 743 801 B1**
(45) Veröffentlichungstag und Bekanntmachung des Hinweises auf die Patenterteilung: **11.03.2020**
(21) Anmeldenummer: 13197254.9
(22) Anmeldetag: 13.12.2013
(51) Int. Cl.: G06F 3/01, G06F 3/0346, B25J 13/02, A61B 34/00, A61B 34/30

(54) **Griffelement und Greifereingabemodul für ein haptisches Eingabesystem**
Handle element and input gripper module for a haptic input system
Poignée et module de préhension pour un dispositif d'entrée haptique

(30) Priorität: 13.12.2012 DE 102012112247
(43) Veröffentlichungstag der Anmeldung: 18.06.2014
(73) Patentinhaber: Karl Storz SE & Co. KG, 78532 Tuttlingen (DE)
(72) Erfinder: Abri, Omid, 10129 Berlin (DE); Schrader, Stephan, 14532 Kleinmachnow (DE); Forster, Jonas, 13595 Berlin (DE)
(74) Vertreter: Witte, Weller & Partner Patentanwälte mbB

(56) Entgegenhaltungen:
- JP-A- H02 284 888
- US-A1- 2010 225 209
- US-A1- 2011 050 405

## Beschreibung

Die vorliegende Erfindung betrifft ein Griffelement zur Anordnung an einem Adapterelement, bzw. ein Adapterelement zur lösbaren Aufnahme eines Griffelements, jeweils um ein Greifereingabemodul für ein haptisches Eingabesystem zur Steuerung von zumindest einem Objekt zu bilden. Ferner betrifft die vorliegende Erfindung ein entsprechendes Greifereingabemodul, ein haptisches Eingabesystem und ein medizinisches Instrumentensystem.

Haptische Eingabesysteme kommen im industriellen und medizinischen Bereich, aber auch im Endverbraucherbereich, zum Einsatz, um die Bewegung eines Benutzers, insbesondere von zumindest einer Hand, auf ein anderes System zu übertragen. Diese anderen Systeme können entweder direkt ansteuerbare Vorrichtungen, wie zum Beispiel medizinische Instrumente, Maschinen, fernsteuerbare Fahrzeuge, Roboter oder dergleichen sein. Alternativ können die durch die Eingabe in diese haptischen Eingabesysteme erhaltenen Informationen auch in (rein) rechnerbezogenen Anwendungen, wie zum Beispiel bei CAD-Anwendungen oder virtueller Realität, umgesetzt werden.

Üblicherweise zeigen diese haptischen Eingabesysteme unterschiedliche Kombinationen an Freiheitsgraden und Achsen, innerhalb derer eine entsprechende Bewegung stattfinden kann. Hierbei sind zumeist drei translatorische und drei rotatorische Freiheitsgrade zu finden. Diese Freiheitsgrade können jeweils mit oder ohne Force-Feedback versehen sein. Mit Force-Feedback sind hier entweder Rückmeldungen einer zu steuernden Vorrichtung oder Rückmeldungen eines Rechnersystems gemeint, die die entsprechende Vorrichtung in der Realität oder das Computersystem in der virtuellen Realität erfährt und über diese Force-Feedback-Rückkopplung auf den Benutzer des haptischen Eingabesystems weitergibt. Neben diesen genannten sechs Freiheitsgraden der räumlichen Bewegung können ferner noch weitere Funktionen an dem haptischen Eingabesystem dem Benutzer zur Verfügung gestellt werden. Eine solche beispielhafte Funktion ist eine Greiferfunktion.

Bei dieser so genannten Greiferfunktion handelt es sich um eine Betätigung einer zu steuernden Vorrichtung (in reeller oder virtueller Realität), die durch eine Greifbewegung des jeweiligen Benutzers mit seiner Hand erreicht wird. Mit Greifbewegung ist in diesem Zusammenhang eine solche Bewegung zu verstehen, in der ein Benutzer zumindest zwei seiner Finger an einer Hand aufeinander zu bewegt, wobei zumindest einer dieser zumindest zwei Finger in der Regel ein Daumen ist.

Zur Erfassung von Bewegungen eines Fingers eines Benutzers beschreibt US 7,480,600 B2 eine Vorrichtung, in der ein Fingerhut auf die Fingerspitze eines Benutzers gesetzt wird. Dieser Fingerhut ist über eine Aufhängungsvorrichtung so mit der dort beschriebenen Vorrichtung verbunden, dass translatorische Bewegungen der Fingerspitze im Raum detektiert werden können. Ferner ist auch die Übermittlung eines Force-Feedbacks auf den jeweiligen Finger beschrieben. Um eine Greifbewegung eines Benutzers detektieren zu können, schlägt die US 7,480,600 B2 vor, eine Mehrzahl der beschriebenen Vorrichtungen zu kombinieren und somit beispielsweise drei Finger einer Hand über eine entsprechende Kombinationsvorrichtung in ihrer Bewegung zu verfolgen. Dies bedeutet, dass jeder zu verfolgende Finger der entsprechenden Hand des Benutzers mit einem entsprechenden Fingerhut versehen wird, der jeweils dann über eine eigene Aufhängevorrichtung mit jeweils einer der dort beschriebenen Vorrichtungen verbunden ist.

Abgesehen von der vergleichsweise aufwändigen Prozedur, jeden Finger einzeln mit einem Fingerhut versehen zu müssen, führt der Aufbau der in der US 7,480,600 B2 beschriebenen Kombinationsvorrichtung dazu, dass ein aufwändiges System an Gestängen vorliegt. Dieses System wird mit zunehmender Anzahl von zu erkennenden Fingern stetig komplizierter, so dass bereits bei drei oder mehr Fingern auch konstruktive Probleme auftreten können.

Die US 7,411,576 B2 beschreibt ein haptisches Interface, das insbesondere zur Bewegung und somit Steuerung entsprechend der zuvor genannten sechs Freiheitsgrade geeignet ist. Dieses haptische Interface weist eine Benutzerschnittstelle auf, die aus einer Nase und einem lösbaren Benutzerverbindungsabschnitt besteht. Dieser lösbare Benutzerverbindungsabschnitt kann unterschiedlich ausgestaltet sein und somit verschiedene Griffformen aufweisen. Insbesondere ist eine stiftartige Form beschrieben. Bei den beschriebenen Formen sind ferner Funktionstasten an der stiftartigen Ausgestaltung vorgesehen, die zu einer Steuerung des haptischen Eingabesystems dienen können. Diese Funktionstasten sind als gewöhnliche Knöpfe ausgestaltet, die bei Bedarf durch einen Finger des Benutzers gedrückt werden können.

Die Steuerung einer Greifbewegung ist nicht durch die US 7,411,576 B2 vorgesehen. Die im Übrigen beschriebenen Funktionstasten dienen lediglich zur Veränderung der Betriebsweise des haptischen Eingabesystems und sind zudem so angeordnet, dass eine ergonomische Betriebsweise des haptischen Eingabesystems der genannten US 7,411,576 B2, wie es zum Beispiel bei der Bedienung von Schneidwerkzeugen durch ein solches haptisches Eingabesystem gewünscht sein könnte, nicht möglich ist.

Auch die US 2005/0043719 A1 beschreibt ein haptisches Eingabesystem mit einem Griffteil mit separaten Eingabeknöpfen. Diese Eingabeknöpfe sind, vergleichbar mit der US 7,411,576 B2, mit verschiedenen Funktionen belegbar.

Die US 2008/0154246 A1 beschreibt ebenfalls ein System zur Durchführung robotergesteuerter chirurgischer Eingriffe. Hierfür ist an einer Handhabe, die wie in der US 7,411,576 B2 im Wesentlichen stiftartig ausgestaltet ist, vorgesehen, dass ein entsprechender Benutzer zwei zueinander verschwenkbar angeordnete Greifelemente zusammendrückt, um bei dem zu steuernden Objekt ein entsprechend ähnliches Zusammenbewegen der Greif- oder Schneidelemente hervorzurufen. Dazu sind diese Griffelemente fest an der Handhabe angeordnet. Ferner sieht die US 2008/0154246 A1 vor, dass ein entsprechender Benutzer, der die Griffelemente zur Betätigung der Greif- oder Schneidwerkzeuge, die gesteuert werden sollen, betätigt, ungefähr ein Gefühl dafür bekommt, wie stark ein entsprechendes Greifwerkzeug oder Schneidwerkzeug betätigt wird. Dies ist in dieser US 2008/0154246 A1 dadurch umgesetzt, dass elastische, im Speziellen auch federnde Elemente vorgesehen werden, die beispielsweise auch in unterschiedlichen Abschnitten eine unterschiedliche Elastizität aufweisen. Hierdurch soll dem Benutzer ein Übergang von einem beispielsweise weiten Öffnungsbereich zu einem engeren Öffnungsbereich eines entsprechenden Werkzeugs vermittelt werden.

Ebenso wie bei der US 7,411,576 B2 liegt auch hier ein eher unergonomisches Greifen der Handhabe vor. Die an dieser Handhabe angeordneten Griffelemente sind aufgrund des Vorsehens mit diesen jeweils mechanischen Bauteilen ferner dahingehend nachteilig, dass diese elastischen/mechanischen Bauteile einer gewissen Abnutzung unterliegen, was ein Auswechseln von Zeit zu Zeit notwendig macht. Aufgrund der festen Integration in die Handhabe in dieser Vorrichtung ist dies jedoch mit einem größeren Aufwand verbunden. Neben der vergleichsweise schlechten ergonomischen Ausgestaltung und den zuvor genannten Verschleißaspekten ist aufgrund der dargestellten Ausführung der Handhabe ferner ein vergleichsweise kompliziertes Reinigen/Sterilisieren notwendig, wenn die Vorrichtung in der Nähe einer Operationsstelle verwendet werden soll. Eine Anwendung eines allgemeinen Überzugs würde hier auch keine Abhilfe schaffen, da dies abermals den Benutzerkomfort einer solchen Vorrichtung herabsetzt.

Die US 2008/0167662 A1 beschreibt ein Kontrollsystem zur Steuerung eines Roboters, insbesondere für chirurgische Eingriffe. Hierfür sieht die Vorrichtung verschiedene Steuervorrichtungen, beispielsweise Joysticks, vor, um das entsprechende zu steuernde Instrument anzusteuern. Um ein entsprechendes haptisches Feedback vom zu steuernden Instrument auf den Benutzer zu übertragen, schlägt die US 2008/0167662 A1 vor, dass ein entsprechender Benutzer einen taktilen Handschuh verwendet. Dieser Handschuh ist so ausgestaltet, dass er entsprechende aufblasbare Elemente enthält, die auf eine Hand eines jeweiligen Benutzers die jeweiligen Kräfte übertragen sollen, die er auch erfahren würde, wenn er das entsprechende Instrument direkt betätigt. Mit anderen Worten bietet die Vorrichtung der US 2008/0167662 A1 ein übliches Kontrollsystem mit den entsprechenden Steuervorrichtungen, deren Nachteile bereits zuvor erwähnt wurden, und integriert hier ein Feedbacksystem in Form eines Handschuhs.

Wenngleich der vorgeschlagene Handschuh eine Optimierung hinsichtlich der Feedbackfunktionen eines solchen Systems bietet, verbleiben hinsichtlich der Ergonomie und sicheren Bedienbarkeit eines entsprechenden Kontrollsystems ferner die üblichen Eingabegeräte. Die zuvor genannten Nachteile von unsicherer Bedienung, möglichem Abrutschen, und schwieriger Kontrolle aufgrund ungünstiger ergonomischer Verhältnisse werden auch nicht durch die Verwendung eines solchen taktilen Handschuhs beseitigt.

Die zuvor exemplarisch beschriebenen haptischen Eingabesysteme ermöglichen zwar rein theoretisch die Übermittlung von Greifinformationen an ein Zielsystem, dies aber zum Beispiel bei der US 7,480,600 B2 durch eine freischwebende Haltung der Hand und durch aufwändige Verbindung der Hand mit den einzelnen haptischen Eingabesystemen für jeden Finger einzeln, oder bei den US 7,411,576 B2 und US 2008/0154246 A1 durch eine eher stiftförmige Anordnung, die ebenfalls für eine gezielte kontrollierte greifartige Bewegung ungeeignet ist. In beiden Vorrichtungen kann aufgrund der vergleichsweise unergonomischen Betätigungsweise leicht ein Ab- bzw. Verrutschen von der eigentlichen Position auftreten, was gerade in schwierigeren Anwendungen, wie zum Beispiel einem operativen Eingriff oder komplizierten mechanischen Eingriffen, fatal sein kann. Dies gilt auch für das System der US 2008/0167662 A1, die im Wesentlichen auf die üblichen Eingabegeräte zu Steuerzwecken zurückgreift. Darüber hinaus stellen die zuvor beschriebenen Vorrichtungen die Verbindung mit dem Benutzer über fest an den Vorrichtungen angeordnete Elemente, wie zum Beispiel in den US 7,480,600 B2, US 2008/0154246 A1 und US 2008/0167662 A1, oder über zumindest vergleichsweise aufwändig gefertigte und auch nicht für eingehende Reinigungen geeignete Elemente, wie in der US 7,411,576 B2, her. Damit sind diese Vorrichtungen für Einsätze im medizinischen Bereich, insbesondere bei chirurgischen Eingriffen nahe der Operationsstelle, unvorteilhaft.

US 2010 0 225 209 A1 offenbart ein Bediensystem für ein chirurgisches Robotersystem. Bei dem System ergreift der Operateur mit seiner linken und rechten Hand jeweils eine Fernbedienung. Die Fernbedienung weist zwei Hebel auf, mittels derer eine Schließbewegung der Hand in eine Betätigung eines Aktuators des Robotersystems umgesetzt werden kann. Es besteht ferner die Möglichkeit, die Fernbedienung zu drehen und zu verschwenken, um das Robotersystem entsprechend zu positionieren.

JP H02-284888 A zeigt ein druckempfindliches Gummimaterial, mit dem die Kraft gemessen werden kann, die ein Benutzer mit seinem Finger aufbringt.

Der vorliegenden Erfindung lag daher die Aufgabe zugrunde, ein Griffelement und ein entsprechendes Adapterelement, die zusammen ein Greifereingabemodul bilden, das Bestandteil eines haptischen Eingabesystems zur Steuerung von zumindest einem Objekt ist, bereitzustellen, die eine ergonomische Betätigung eines haptischen Eingabesystems insbesondere im Hinblick auf Greifbewegungen, oder greifähnliche Bewegungen, ermöglichen, wobei dabei insbesondere für den Benutzer eine sichere Kontrolle und Bewegung ermöglicht werden soll. Ferner soll die Vorrichtung vorzugsweise auch einfach zu reinigen, insbesondere an den offen liegenden Griff- und Kontaktbereichen mit einem Benutzer einfach zu sterilisieren sein.

Diese Aufgabe wird zum einen durch ein Griffelement zur Anordnung an einem Adapterelement, um ein Greifereingabemodul für ein haptisches Eingabesystem zur Steuerung von zumindest einem Objekt zu bilden, gelöst, mit
- zumindest einer Aufnahme zur Aufnahme von zumindest zwei Fingern eines Benutzers darin, wobei die Aufnahme zumindest in einem Abschnitt in Wirkverbindung mit zumindest einem Sensormittel steht, und
- zumindest einem Verbindungselement zur Anordnung des Griffelements an dem Adapterelement,
wobei die Aufnahme so ausgestaltet ist, dass eine Bewegungsinformation einer Bewegung von zumindest einem Finger des Benutzers in der Aufnahme durch das Sensormittel detektierbar und damit die Bewegungsinformation zur Steuerung des zumindest einen Objekts übertragbar ist.

Durch Aufteilung des Greifereingabemoduls in ein Griffelement und ein Adapterelement wird die Möglichkeit geschaffen, ein entsprechendes haptisches Eingabesystem so auszugestalten, dass ein fester Teil, beispielsweise durch Eindecken mit einer Schutzhülle oder ständige Sterilisierung, steril gehalten werden kann, während ein auswechselbarer Teil, nämlich das Griffelement, zur Sterilisierung, beispielsweise in einem Autoklaven oder anderen Sterilisiervorrichtungen, entfernt werden kann. Daneben kann das Griffelement auch als (steriles) Einwegelement vorgesehen sein.

Ein weiterer Vorteil dieser Ausgestaltung ist die Möglichkeit, dass ein jeweiliges Griffelement für einen bestimmten Zweck bzw. eine bestimmte Anwendung in medizinischen oder anderen Steuerungsanwendungen vorgesehen werden kann. So kann in Abhängigkeit der gewünschten Steuerung jeweils die Zahl der Aufnahmen und/oder die Zahl der Finger pro Aufnahme unterschiedlich sein und durch Auswechslung eines jeweiligen Griffelements angepasst werden. Die Anordnung des Griffelements an dem Adapterelement erfolgt dabei vorzugsweise kraftschlüssig, formschlüssig, stoffschlüssig oder in jeder beliebigen Kombination dieser Verbindungsarten.

Durch die zumindest eine Aufnahme ist eine feste Position für die jeweiligen Finger eines Benutzers vorgesehen. Hierbei ist unter zumindest einer Aufnahme für zumindest zwei Finger sowohl ein durchgehender als auch ein durch beispielsweise Stege unterbrochener Bereich zu verstehen. Ein entsprechender Finger eines Benutzers befindet sich ferner dann "in der Aufnahme", wenn er an dem für seine Positionierung vorgesehenen Platz angeordnet ist. Dabei kann es sich sowohl um einfache Mulden handeln, die den Finger aufnehmen können, als auch in einer bevorzugten Variante um Öffnungen im Griffelement handeln, die in etwa die Größe und/oder Form des Fingers aufweisen und diesen zumindest teilweise zu den Seiten oder rundherum umgeben. Eine Bewegung "in der Aufnahme" schließt dementsprechend eine Bewegung des Fingers im Rahmen des freien Spiels des Fingers in dieser Aufnahme als auch eine Deformation des die Aufnahme begrenzenden bzw. eingrenzenden Materials mit ein.

Dadurch, dass der entsprechende Finger bzw. die entsprechenden Finger eines Benutzers in zumindest einer Aufnahme aufgenommen sind, ist ein sicherer Halt und somit eine sichere und zuverlässige Bedienung eines entsprechenden haptischen Eingabesystems gewährleistet. Durch die Detektion von Bewegungen in der Aufnahme ist ferner gewährleistet, dass der Benutzer auch den Bereich der Aufnahme, das heißt den Bereich, der ihm durch die Aufnahme zur Verfügung gestellt wird, mit seinen Fingern nicht verlassen muss. Dies trägt ferner zu der sicheren und zuverlässigen Bedienung des haptischen Eingabesystems bei. Eine Bewegung des Fingers, beispielsweise um einen Greifvorgang durchzuführen, die durch eine Greifbewegung der Finger umgesetzt wird, kann somit sehr ergonomisch realisiert werden. Der Benutzer greift dabei zum Beispiel einfach mit seinen Fingern in das Griffelement hinein, hat somit einen festen Griff an dem haptischen Eingabesystem zur räumlichen Steuerung von zumindest einem Objekt, und kann dann durch Greifbewegungen gleichzeitig und ohne Verlust des festen Griffs am haptischen Eingabesystem ein Greifen, Schneiden oder eine sonstige vergleichbare Aktuation an dem zu steuernden Objekt auslösen. Dies unterscheidet die vorliegende Erfindung in vorteilhafter Weise von dem eingangs genannten Stand der Technik.

Die Möglichkeit der Detektion der zugehörigen Bewegungsinformation durch die Bewegung des zumindest einen Fingers des Benutzers in der Aufnahme durch das entsprechende Sensormittel schließt sowohl Fälle mit ein, bei denen das Sensormittel in dem Griffelement vorliegt, als auch solche, wo das Sensormittel an dem Adapterelement oder einem mit diesem in Wirkverbindung stehenden Element vorliegt. Ein entsprechender Sensor kann somit, wie dies im Folgenden noch ausgeführt wird, entweder in dem Griffelement, dem Adapterelement oder einer externen Vorrichtung, die auch Bestandteil des haptischen Eingabesystems sein kann, vorliegen. Die vorzugsweise kraft-, form- und/oder stoffschlüssige Anordnung des Griffelements an dem Adapterelement garantiert ferner, dass die Bewegungen in den zuvor genannten maximal sechs Freiheitsgraden im haptischen Eingabesystem gewährleistet werden können, wenn der Benutzer das haptische Eingabesystem über das Griffelement steuert.

Der Begriff "Bewegungsinformation" meint im Übrigen in diesem Zusammenhang jegliche Information, die aus einer Bewegung eines entsprechenden Fingers gewonnen werden kann, wie z.B. Richtung, Geschwindigkeit, Kraft, Druck, Position und Dauer. Diese Bewegungsinformation kann dann zur Steuerung des zu steuernden Objekts direkt oder mittelbar an dieses übertragen werden. Dabei schließt "Bewegung" im Zusammenhang mit dieser Erfindung sowohl eine tatsächliche Bewegung, dass heißt eine Veränderung der Positionen des jeweiligen Fingers/der jeweiligen Finger, als auch eine nicht (komplett) ausgeführte Bewegung mit ein, bei der beispielsweise eine entsprechende Kraftausübung des jeweiligen Finers/der jeweiligen Finger auf einen starren oder nur gering veränderbaren Widerstand trifft, der somit die tatsächliche Bewegung unterbindet. Mit anderen Worten würde die tatsächliche Bewegung stattfinden, wenn sie nicht durch den Widerstand behindert werden würde. So kann beispielsweise eine solche Kraftausübung dann entsprechend als Bewegungsinformationen im Sinne der vorliegenden Erfindung detektiert werden.

In eine Ausgestaltung des Griffelements weist das Griffelement einen kugelförmigen Körper auf, wobei eine kugelförmige Oberfläche des Körpers mindestens eine Öffnung für die zumindest eine Aufnahme aufweist.

In einer weiteren Ausgestaltung des Griffelements weist das Griffelement einen Körper mit einem Verbindungselement in Form einer Ausnehmung auf, wobei das Verbindungselement für eine Anordnung des Griffelements an einem Adapterelement ausgebildet ist. Bevorzugt handelt es sich bei dem Körper um einen kugelförmigen Körper, wobei eine kugelförmige Oberfläche des Körpers Öffnungen für die Aufnahme aufweist.

In einer weiteren Ausgestaltung des Griffelements der vorliegenden Erfindung ist das Griffelement im Wesentlichen aus einem elastischen Material hergestellt und/oder wird die Wirkverbindung mit dem zumindest einen Sensormittel durch ein elastisches Material realisiert.

Die Ausgestaltung des gesamten Griffelements durch ein elastisches Material hat zum einen den Vorteil, dass dies ebenfalls zur Ergonomie des gesamten Gerätes beiträgt. Die Form des Griffelements kann somit geringfügigen Unterschieden in den Formen der Hände verschiedener Benutzer des haptischen Eingabesystems angepasst werden. Ebenso trägt ein elastisches Material als Griffelement positiv zum Benutzerkomfort bei.

Die Ausgestaltung hat ferner, ebenso wie die Ausgestaltung, dass die Wirkverbindung mit dem zumindest einen Sensormittel durch ein elastisches Material realisiert wird, den Vorteil, dass der Benutzer so nicht direkt an den Sensor oder ein allgemeines Sensormittel, wie zum Beispiel einen Taster, einen Druckmesser, etc., fassen muss. Dies ermöglicht die Ausgestaltung von haptischen Eingabesystemen, die insbesondere Griffelemente mit geschlossenen und auch glatten Oberflächen aufweisen, die somit einfacher zu reinigen sind. Dabei kann ein entsprechendes Sensormittel entweder direkt unter der Oberfläche der Aufnahme angeordnet sein und eine Kraftübertragung durch ein elastisches Material realisiert werden, als auch beispielsweise im Adapterelement angeordnet sein, wobei auch hier eine Übermittlung der Bewegung, in Form von zum Beispiel einer Kraftübermittlung, durch das elastische Material und über die Wirkverbindung zwischen Griffelement und Adapterelement in das Adapterelement erfolgen kann.

In einer weiteren Ausgestaltung des Griffelements der vorliegenden Erfindung ist die zumindest eine Aufnahme so ausgestaltet, dass eine Bewegung von zumindest einem Finger des Benutzers in der zumindest einen Aufnahme über das Verbindungselement durch zumindest ein Sensormittel, das an dem Adapterelement angeordnet ist, detektierbar ist.

In dieser Ausgestaltung liegt somit das Sensormittel auf Seiten des Adapterelements vor. Das Griffelement, beispielsweise in Form des ihn aufbauenden Materials, dient somit als Wirkverbindung zwischen zumindest einem Finger des Benutzers und dem Sensormittel. Dies wird durch die zuvor genannte Wirkverbindung zwischen Griffelement und Adapterelement ermöglicht.

Diese Ausgestaltung hat den Vorteil, dass das Griffelement so ohne Sensormittel, das heißt zum Beispiel ohne Sensorelektronik oder dergleichen, auskommt. Solch ein Griffelement kann daher relativ einfach und unkompliziert gefertigt werden. Dies ermöglicht einerseits, dass das Griffelement einfach gereinigt bzw. sterilisiert werden kann, da eine Gefährdung von komplexerer oder empfindlicher Elektronik durch zum Beispiel aggressive Chemikalien oder hohe Temperaturen, wie sie zum Beispiel in Autoklaven vorkommen, nicht vorliegt. Andererseits ist so auch eine Ausgestaltung des Griffelements als (steriler) Einmalartikel möglich, da durch die Abwesenheit von komplizierterer oder aufwändigerer Elektronik und sonstigen Mess- bzw. Sensorelementen, oder allgemein Sensormittel, die Herstellung deutlich günstiger und einfacher ausfallen kann, so dass die Verwendung als Einmalartikel wirtschaftlich infrage kommt.

In einer weiteren Ausgestaltung des Griffelements der vorliegenden Erfindung weist das Griffelement zumindest ein Sensormittel zur Detektion von zumindest einer Bewegung von zumindest einem Finger des Benutzers in der Aufnahme auf.

Bei dieser Ausgestaltung liegt nun ein Sensormittel, vorzugsweise ein Sensor, in dem Griffelement vor. Dieses Sensormittel kann nun direkt in der Aufnahme, als auch an einer anderen Stelle des Griffelements, die in Wirkverbindung mit der Aufnahme steht, angeordnet sein. Letzteres ist beispielsweise aufgrund des Materials, aus dem das Griffelement besteht, möglich. Dadurch kann die Bewegung des zumindest einen Fingers des Benutzers in der Aufnahme detektiert und zum Beispiel in elektrische Signale umgewandelt werden, um letztendlich eine Steuerung des zumindest einen Objekts zu ermöglichen.

Durch Vorsehen des zumindest einen Sensormittels in dem Griffelement wird ermöglicht, dass das entsprechende Griffelement mit den Sensormitteln auf einfache Weise ausgetauscht werden kann. Dies ist von Vorteil, wenn zum Beispiel ein oder mehrere Sensormittel defekt sind und ausgetauscht werden müssen. Dies bedeutet bei anderen Vorrichtungen normalerweise eine aufwändige Reparatur. Aufgrund der vorliegenden Ausgestaltung genügt es hingegen jedoch, lediglich das Griffelement auszutauschen. Dies kann beispielsweise insbesondere dann von Vorteil sein, wenn die jeweiligen Sensormittel vergleichsweise einfache und kostengünstige Sensoren sind und eine sonstige Reparatur deutlich kostspieliger wäre als das Bauteil an sich. Ferner ist bei solch einer Ausgestaltung auch denkbar, eine Verwendung des Griffelements als Einwegartikel vorzusehen. Dies hängt jedoch maßgeblich von der bestimmten Verwendung und wirtschaftlichen Aspekten ab.

In einer weiteren Ausgestaltung des Griffelements der vorliegenden Erfindung weist das zumindest eine Sensormittel zumindest einen Sensor, vorzugsweise zumindest einen Drucksensor, einen Kraftsensor oder einen Bewegungssensor, auf.

Durch die Verwendung dieser Arten von Sensoren ist es möglich, die Bewegung eines entsprechenden Fingers in der Aufnahme, insbesondere durch Detektion eines Drückens des Fingers in oder auf das Material des Griffelements, zu detektieren. Ferner können neben reinen Druckdetektionen auch Spannungen oder Bewegungen des Materials detektiert werden, so dass beispielsweise auch Scherkräfte erfasst werden können. Dies ermöglicht, je nach gewünschtem Anwendungsgebiet, eine sehr detaillierte Detektion von den Bewegungsinformationen des zumindest einen Fingers in der Aufnahme.

In einer weiteren Ausgestaltung des Griffelements der vorliegenden Erfindung ist die zumindest eine Aufnahme ferner so ausgestaltet, dass sie eine Übermittlung zumindest einer Force-Feedback-Information zu zumindest einem Finger des Benutzers, der in der zumindest einen Aufnahme aufgenommen ist, ermöglicht.

Die Übermittlung von Force-Feedback-Informationen führt dazu, dass ein Benutzer einen haptischen Eindruck von der Einsatzstelle des zu steuernden Objekts bekommt, idealerweise so, als wenn er direkt an dem zu steuernden Objekt arbeiten würde und dadurch selber die haptischen Eindrücke erfährt. "Force-Feedback" meint in diesem Zusammenhang somit zum einen die haptischen Eindrücke, die sich durch das zu steuernde Objekt direkt ergeben, wie zum Beispiel Widerstand beim Betätigen einer Klemme oder Schere, als auch in solche haptischen Rückkopplungen transformierte Hinweise, wenn zum Beispiel eine Warnung oder eine sonstige Information an den Benutzer übermittelt werden soll, die insbesondere dessen Aufmerksamkeit erfordern. Force-Feedback kann in diesem Zusammenhang unterschiedlich ausgestaltet sein. So kann man zum einen beispielsweise tatsächliche Widerstände oder Gegenkräfte beim Greifen als auch andere Aufmerksamkeit erzeugende Aktionen, wie zum Beispiel Vibrationen, darunter verstehen.

Das Vorsehen von Force-Feedback-Informationen, bzw. deren Übermittlung zu zumindest einem Finger des Benutzers, hat damit den Vorteil, dass der Benutzer einerseits so auf unterschiedliche Gegebenheiten am zu steuernden Objekt selber oder an dem gesamten Betätigungs- bzw. Operationsvorgang aufmerksam gemacht werden kann. Durch die Übermittlung über die Aufnahme an sich ist es ferner möglich, dass entsprechende Vorrichtungen, wie zum Beispiel Aktuatoren, nicht direkt mit dem Benutzer in Kontakt treten müssen, sondern dass auch eine Vermittlung, bzw. Übermittlung, durch beispielsweise das Material des Griffelements möglich ist.

In einer weiteren Ausgestaltung des Griffelements der vorliegenden Erfindung ist die zumindest eine Aufnahme so ausgestaltet, dass die zumindest eine Force-Feedback-Information von dem Adapterelement über das Verbindungselement an zumindest einen Finger des Benutzers, der in der zumindest einen Aufnahme aufgenommen ist, weitergebbar ist.

Bei dieser Ausgestaltung kann, vergleichbar zu der parallelen Ausgestaltung mit dem Sensormittel im Adapterelement, wie zuvor bereits beschrieben, jegliche Technik, wie in diesem Fall beispielsweise Aktuatoren, in dem Adapterelement vorgesehen sein. Das bedeutet, dass das Griffelement selber als ein entweder einfach zu sterilisierendes oder sogar als Einmalprodukt gefertigt bzw. bereitgestellt werden kann. Dies trägt entsprechend dazu bei, dass hygienische Vorschriften, wie sie zum Beispiel bei chirurgischen Operationen vorliegen, leicht einzuhalten sind. Je nach verwendetem Material für das Griffelement kann auch eine gewisse Abnutzung durch die Benutzung durch einen Benutzer auftreten. Somit kann auch, unabhängig von chirurgischen Eingriffen, generell ein leichter und kostengünstiger Austausch von Griffelementen stattfinden, so dass ein neues Griffelement regelmäßig an einem haptischen Eingabesystem vorgesehen werden kann. Dies erhöht ferner den Komfort für die Benutzer.

In einer weiteren Ausgestaltung des Griffelements der vorliegenden Erfindung weist das Griffelement zumindest einen Aktuator auf, über den zumindest eine Force-Feedback-Information an zumindest einen Finger des Benutzers, der in der zumindest einen Aufnahme aufgenommen ist, weitergebbar ist.

Bei dieser Ausgestaltung wird die Force-Feedback-Information somit direkt durch das Griffelement, bzw. durch Aktuatoren, die in diesem angeordnet sind, bereitgestellt. Eine Ankopplung an das gesamte System kann hier, wie auch bei den zuvor beschriebenen Sensoren bzw. Sensormittel, entweder direkt an dem Griffelement, oder vermittelt über das Adapterelement erfolgen. Für letztere Variante würden das Adapterelement und das Griffelement dann jeweils entsprechende Kontaktverbindungsstellen vorsehen.

Durch Vorsehen der Aktuatoren in dem Griffelement ist es auch hier möglich, einfache Reparaturen bei dem Vorliegen von Fehlfunktionen durchzuführen. Dies ist insbesondere dann attraktiv, wenn es sich bei den Aktuatoren um vergleichsweise günstige Bauteile handelt, bei denen eine aufwändige Reparatur in einer wiederzuverwendenden Vorrichtung den Rahmen der Bauteilkosten überschreiten würde. Abhängig von der Art und den Kosten für den Aktuator kommt auch hier eine zuvor beschriebene Verwendung als einfach zu sterilisierendes bzw. Einwegprodukt infrage. Aktuatoren können dementsprechend beliebige elektrische oder mechanische Vorrichtungen sein, die eine Vibration oder einen Druck auf den zumindest einen Finger des Benutzers ausüben. Dies kann entweder direkt, das heißt durch oder aus dem Material des Griffelements heraus, geschehen, oder durch das Material des Griffelements vermittelt sein. Mit anderen Worten können die Aktuatoren somit so angeordnet sein, dass sie direkt mit dem zumindest einen Finger des Benutzers in Berührung kommen oder aber durch ein Material des Griffelements von diesem getrennt sind. In letzterem Fall ist abermals die Verwendung eines elastischen Materials, entweder für das gesamte Griffelement oder für den Bereich der Aufnahme, das heißt den Kontaktbereich mit dem zumindest einen Finger des Benutzers, von Vorteil. Neben den zuvor beschriebenen elektrischen oder mechanischen Vorrichtungen können beispielsweise auch Hohlkammern als Aktuatoren vorgesehen sein. Diese können zum Beispiel durch ein Fluid, wie zum Beispiel ein Gas oder eine Flüssigkeit, in ihrem Volumen und Druck verändert werden. Dies kann bei entsprechender Anordnung im Griffelement dann zu einem Druck, oder zumindest Gegendruck, auf den zumindest einen Finger des Benutzers führen. Gerade die Verwendung dieser zuletzt genannten Aktuatoren in Form von Hohlkammern stellt eine vergleichsweise günstige Variante von Aktuatoren dar. Entsprechend kämen auch solche Ausgestaltungen als einfach zu sterilisierende oder Einwegprodukte infrage. Die Anbindung eines solchen Aktuators erfolgt dann, analog zu elektrischen, oder gegebenenfalls mechanischen, Verbindungen der zuvor beschriebenen Aktuatoren, entweder über Verbindung in Form von beispielsweise Schläuchen direkt mit dem Griffelement zu dem jeweiligen Aktuator oder vermittelt über das Adapterelement.

In einer weiteren Ausgestaltung des Griffelements der vorliegenden Erfindung weist das Griffelement zumindest ein passives Force-Feedback-Element auf.

Im Gegensatz zu den zuvor beschriebenen aktiven Force-Feedback-Elementen, die durch eine äußere Steuerinformation, beispielsweise einen elektrischen Impuls oder die Erhöhung des Drucks in einem Hohlraum durch ein Fluid, eine Force-Feedback-Information auf den Finger des Benutzers übertragen, weisen passive Force-Feedback-Elemente insbesondere Elemente auf, die dazu geeignet sind, ihre Materialeigenschaften zu ändern. Hierbei ist mit Materialeigenschaften vorzugsweise die Elastizität oder der Widerstand gegen eine Stauchung gemeint. Mit anderen Worten meint ein passives Force-Feedback-Element im Rahmen dieser Erfindung ein Element, welches Eigenschaften aufgrund eines Steuersignals so verändern kann, dass sie durch einen Benutzer fühlbar sind. Konkret könnte somit beispielsweise ein passives Force-Feedback-Element in dem Griffelement-Material angeordnet sein, so dass der Benutzer durch eine Greifbewegung (oder eine Öffnungsbewegung der Hand) entweder direkt oder vermittelt durch das Material des Griffmaterials auf dieses Force-Feedback-Element drückt. Liegt nun bei einer Steuerung des zu steuernden Objekts beispielsweise ein größerer Widerstand vor, kann der Widerstand gegen Stauchung bzw. die Elastizität des passiven Force-Feedback-Elements so verändert werden, dass auch hier ein festeres Material vorliegt und somit ein vergleichbarer haptischer Eindruck für den Benutzer entsteht. Umgekehrt kann dieses Material in seiner Elastizität bzw. im Widerstand gegen Stauchung so verändert werden, dass ein weiches Material simuliert wird, wenn dieses beispielsweise auch auf Seiten des zu steuernden Objekts vorliegt. Solch eine Anwendung ist zum Beispiel insbesondere bei Greif- oder Schneidvorgängen, die mithilfe des haptischen Eingabesystems ferngesteuert werden sollen, von Vorteil, um dem Benutzer genau den Eindruck von einer entsprechenden Operations- bzw. Betätigungsstelle des zu steuernden Objekts zu vermitteln. Nicht beschränkende Beispiele für solche passiven Force-Feedback-Elemente sind elektrorheologische oder magnetorheologische Fluide, die durch Anwendung eines elektrischen Feldes entweder direkt oder vermittelt durch eine Veränderung eines Magnetfeldes entsprechend den zuvor gemachten Ausführungen verändert werden können.

In einem weiteren Aspekt der vorliegenden Erfindung wird die zuvor genannte Aufgabe ferner durch ein Adapterelement zur lösbaren Aufnahme eines Griffelements, um ein Greifereingabemodul für ein haptisches Eingabesystem zur Steuerung von zumindest einem Objekt zu bilden, gelöst, mit
- einer Schnittstelle zur Kommunikation mit dem haptischen Eingabesystem oder dem zumindest einen Objekt, und
- zumindest einem Aufnahmeelement, zur Aufnahme des Griffelements an dem Adapterelement,
wobei das zumindest eine Aufnahmeelement so ausgestaltet ist, dass das Adapterelement und ein mit diesem verbindbares Griffelement zur Detektion von Bewegungsinformationen von zumindest einem Finger des Benutzers in einer Aufnahme des Griffelements durch ein Sensormittel in Wirkverbindung bringbar sind.

Diese Ausgestaltung des erfindungsgemäßen Adapterelements ermöglicht das entsprechende Gegenstück zu dem zuvor beschriebenen Griffelement der vorliegenden Erfindung. Entsprechend den zuvor gemachten Ausführungen liegt auch hier der Vorteil in der Ausgestaltung, dass die Detektion von Bewegungsinformationen bzw. Bewegungen eines Fingers des Benutzers in einer Aufnahme möglich ist und dass diese Informationen entsprechend übertragen werden können. Diese Übertragung findet bei der Ausgestaltung dieses erfindungsgemäßen Adapterelements über das Aufnahmeelement in das Adapterelement statt. Dies kann entsprechend so sein, dass mechanische Informationen, wie zum Beispiel Kräfte, Druck oder Bewegung, durch ein Medium des Griffelements in das Adapterelement weitergeleitet werden können, oder indem die entsprechenden Bewegungsinformationen zuvor schon in elektrische oder andere Kommunikationssignale umgewandelt und dann über entsprechende Leitungen in das Adapterelement transportiert werden können. Die übrigen Vorteile, die sich aus der Aufnahme des Fingers in der zumindest einen Aufnahme ergeben, wurden bereits im vorhergehenden Zusammenhang mit dem Griffelement beschrieben und sind auf dieses erfindungsgemäße Adapterelement entsprechend übertragbar. Durch die vorgesehene Schnittstelle kann ferner dann eine Kommunikation entweder mit dem haptischen Eingabesystem und über dieses vermittelt oder direkt zu dem zumindest einen Objekt stattfinden. Folglich können somit Informationen an und von der Steuerung des Objekts an das Adapterelement und folglich auch an das Griffelement übertragen werden. Auch hier erfolgt die Anordnung des Griffelements an dem Adapterelement vorzugsweise kraftschlüssig, formschlüssig, stoffschlüssig oder in jeder beliebigen Kombination dieser Verbindungsarten. Das beschriebene Adapterelement, auch in seinen nachfolgend beschriebenen Ausgestaltungen, ist bevorzugt Teil eines Greifereingabemoduls.

In einer Ausgestaltung des Adapterelements der vorliegenden Erfindung weist das Adapterelement zumindest ein Sensormittel zur Detektion von zumindest einer Bewegung von zumindest einem Finger des Benutzers auf.

Entsprechend dieser Ausgestaltung sieht das Adapterelement selber ein Sensormittel vor. "Sensormittel" meint in diesem Zusammenhang, dass ein entsprechender Sensor entweder direkt in dem Adapterelement angeordnet ist oder zumindest mit dem Adapterelement in Wirkverbindung steht. Beispielsweise kann ein Druckunterschied durch die Weiterleitung eines Fluids an einen externen Sensor weitergegeben werden, ohne dass der Sensor selber in dem Adapterelement vorliegen muss. Dies versteht sich aber auch als zu der zuvor beschriebenen Ausgestaltung zugehörig und ist insoweit auch auf das Griffelement übertragbar. Durch das Vorsehen dieser Sensormittel, vorzugsweise der Sensoren in dem Adapterelement, bzw. mit diesem in Wirkverbindung stehend, ergibt sich die zuvor beschriebene Möglichkeit, das Griffelement als ein einfach zu sterilisierendes oder als Einwegprodukt bereitzustellen. Außerdem ermöglicht diese Ausgestaltung allgemein das einfache und kostengünstige Auswechseln des Griffelements, was in Anbetracht einer möglichen Abnutzung des Griffelements auch ferner zum Benutzerkomfort beiträgt.

In einer weiteren Ausgestaltung des Adapterelements der vorliegenden Erfindung weist das zumindest eine Sensormittel zumindest einen Sensor und vorzugsweise einen Drucksensor, einen Kraftsensor oder einen Bewegungssensor auf.

Durch die Ausgestaltung mit diesen Sensoren ist es möglich, sowohl Druck- als auch Zugkräfte, die durch den Finger des Benutzers innerhalb der Aufnahme erzeugt werden, als auch Scherkräfte, die durch mögliche Verdrehbewegung des Fingers des Benutzers auftreten können, zu erfassen. Hierzu wird durch das Material des Griffelements entsprechend eine Wirkverbindung zu dem zumindest einen Finger des Benutzers hergestellt, so dass die entsprechenden Zug-, Druck- oder Scherkräfte auf die entsprechenden Sensoren im Adapterelement bzw. über das Adapterelement weitergeleitet werden können.

In einer weiteren Ausgestaltung des Adapterelements der vorliegenden Erfindung ist das Aufnahmeelement so ausgestaltet, dass zumindest eine Force-Feedback-Information zu zumindest einem Finger des Benutzers, der in einer Aufnahme des Griffelements aufgenommen ist, über das Griffelement weitergebbar ist.

Durch diese Ausgestaltung wird, entsprechend der zuvor beschriebenen parallelen Ausgestaltung für das Griffelement, ermöglicht, dass eine Force-Feedback-Information, beispielsweise von dem haptischen Eingabesystem oder von dem zu steuernden Objekt, an einen Finger übermittelt werden kann. Dabei schließt dies sowohl Ausgestaltungen ein, bei denen eine entsprechende Force-Feedback erzeugende Vorrichtung in dem Adapterelement vorliegt, als auch im Griffelement vorliegt. Hierfür sind das Aufnahmeelement des Adapterelements, als auch das entsprechende Verbindungselement des Griffelements, so ausgestaltet, dass entweder die Information von Kräften durch eine Wirkverbindung direkt, das heißt durch Kraft-, Form- und/oder Stoffschluss, übertragen werden kann, als auch, dass eine Übertragung von elektrischen oder auch mechanischen Signalen, wie zum Beispiel Druckunterschiede eines Fluids, übertragen werden kann. Dadurch wird entsprechend den zuvor gemachten Ausführungen im Zusammenhang des Griffelements die Möglichkeit gegeben, dass der Benutzer Force-Feedback-Informationen erhält und somit einen besseren haptischen Eindruck von dem zu steuernden Objekt und dessen Umgebung erhalten kann. Ebenso können Warn- oder andere Hinweisinformationen über ein solches Force-Feedback-System an den Benutzer übermittelt werden.

In einer weiteren Ausgestaltung des Adapterelements der vorliegenden Erfindung weist das Adapterelement zumindest einen Aktuator auf, über den zumindest eine Force-Feedback-Information an zumindest einen Finger des Benutzers, der in einer Aufnahme des Griffelements aufgenommen ist, weitergebbar ist.

In dieser Ausgestaltung ist entsprechend ein Aktuator in dem Adapterelement vorgesehen. Ein solcher Aktuator kann, entsprechend der zuvor gemachten Ausführungen für das Griffelement, ein mechanisch oder elektrisch betriebener Aktuator als auch beispielsweise ein im Volumen veränderbarer Hohlraum sein. Das Vorsehen des Aktuators in dem Adapterelement hat entsprechend abermals den Vorteil, dass das Griffelement so möglichst einfach gehalten wird und für Sterilisation, als Einwegprodukt oder allgemein für ein regelmäßiges Austauschen infrage kommt. Die Kraft bzw. Bewegung oder allgemeine Veränderung, die der Aktuator bei seiner Operation aufweist, werden dann durch das Material des Griffelements oder durch geeignete Verbindungen in diesem Griffelement direkt zu dem Finger des Benutzers übertragen. Hierdurch wird ebenfalls die zuvor erwähnte Wirkverbindung bereitgestellt. Im Übrigen ergeben sich die weiteren Vorteile aus den zuvor gemachten Ausführungen im Rahmen der entsprechenden parallelen Ausgestaltung für das Griffelement.

In einer weiteren Ausgestaltung des Adapterelements der vorliegenden Erfindung weist das Adapterelement zumindest ein passives Force-Feedback-Element auf.

Das passive Force-Feedback-Element ermöglicht, ebenfalls wie zuvor bereits für das erfindungsgemäße Griffelement beschrieben, die Einbindung von besseren haptischen Eindrücken für einen Benutzer des haptischen Eingabesystems. Somit ist eine Übertragung von den lokalen Gegebenheiten am zu steuernden Objekt auf das haptische Eingabesystem, insbesondere auf das Greifereingabemodul, und somit auf den zumindest einen Finger des Benutzers möglich. Weitere Vorteile und Erläuterungen ergeben sich ebenfalls aus dem Zusammenhang der entsprechenden Ausgestaltungen im Zusammenhang des erfindungsgemäßen Griffelements der vorliegenden Erfindung.

In einem weiteren Aspekt der vorliegenden Erfindung wird die zuvor genannte Aufgabe ferner durch ein Greifereingabemodul mit einem Griffelement der vorliegenden Erfindung und einem Adapterelement zur Aufnahme des Griffelements zur Bedienung eines haptischen Eingabesystems zur Steuerung von zumindest einem Objekt gelöst, wobei das Adapterelement eine Schnittstelle zur Kommunikation mit dem haptischen Eingabesystem oder dem zumindest einen Objekt, und zumindest ein Aufnahmeelement zur Aufnahme des Griffelements an dem Adapterelement aufweist und wobei entweder das Griffelement und/oder das Adapterelement mit einem Sensormittel in Wirkverbindung steht.

Diese Ausgestaltung für ein erfindungsgemäßes Greifereingabemodul überträgt die zuvor genannten Ausgestaltungsvarianten der Griffelemente und die damit verbundenen Vorteile auf ein solches erfindungsgemäßes Greifereingabemodul. Dabei weist das Griffelement und/oder das Adapterelement vorzugsweise das Sensormittel auf. Ferner erfolgt auch hier die Anordnung des Griffelements an dem Adapterelement dabei vorzugsweise kraftschlüssig, formschlüssig, stoffschlüssig oder in jeder beliebigen Kombination dieser Verbindungsarten.

In einer Ausgestaltung des Greifereingabemoduls der vorliegenden Erfindung ist das Adapterelement ein Adapterelement der vorliegenden Erfindung.

Diese Ausgestaltung des erfindungsgemäßen Greifereingabemoduls überträgt nun ferner die zuvor beschriebenen Ausgestaltungen des Adapterelements und die damit verbundenen Vorteile auf ein entsprechendes Greifereingabemodul der vorliegenden Erfindung.

In einem weiteren Aspekt der vorliegenden Erfindung wird die zuvor genannte Aufgabe durch ein haptisches Eingabesystem zur Steuerung von zumindest einem Objekt, mit einem Adapterelement der vorliegenden Erfindung, gelöst.

Diese Ausgestaltung des haptischen Eingabesystems mit einem Adapterelement der vorliegenden Erfindung überträgt die zuvor beschriebenen Ausgestaltungsvarianten und die damit jeweils verbundenen Vorteile des erfindungsgemäßen Adapterelements auf ein haptisches Eingabesystem. Dieses steht entsprechend dann zur Benutzung mithilfe von Griffelementen, beispielsweise entsprechend der vorliegenden Erfindung, zur Verfügung.

In einem weiteren Aspekt der vorliegenden Erfindung wird die zuvor genannte Aufgabe ferner durch ein haptisches Eingabesystem zur Steuerung von zumindest einem Objekt, mit einem Greifereingabemodul der vorliegenden Erfindung, gelöst.

Diese Ausgestaltung überträgt entsprechend auch die zuvor beschriebenen Ausgestaltungsvarianten und die damit verbundenen Vorteile des erfindungsgemäßen Greifereingabemoduls auf ein haptisches Eingabesystem. Dies bedeutet, dass entsprechend die Vorteile des erfindungsgemäßen Griffelements, gegebenenfalls in Kombination mit dem erfindungsgemäßen Adapterelement, auf ein solches haptisches Eingabesystem samt deren Ausgestaltungsvarianten übertragen werden.

In einem weiteren Aspekt der vorliegenden Erfindung wird die zuvor genannte Aufgabe ferner durch ein medizinisches Instrumentensystem gelöst, das
- zumindest ein medizinisches Instrument, und
- zumindest ein haptisches Eingabesystem der vorliegenden Erfindung aufweist, wobei das zumindest eine medizinische Instrument durch das haptische Eingabesystem steuerbar ist.

Diese Ausgestaltung eines medizinischen Instrumentensystems bietet nun ebenfalls die zuvor gemachten Ausführungen für das haptische Eingabesystem, das damit verbundene Greifereingabemodul, das Griffelement und auch das Adapterelement in den jeweiligen Ausgestaltungsvarianten mit den damit verbundenen Vorteilen. Ein solches medizinisches Instrumentensystem dient somit zur Operation eines Patienten durch einen Benutzer, das heißt einen Operateur, entweder in der Nähe zum tatsächlichen Operationsbereich oder auch in einer ferngesteuerten Anwendung. Das heißt, dass ein solches medizinisches Instrumentensystem in einer solchen Anwendung auf verschiedene Orte aufgeteilt ist, worin das medizinische Instrument sich am Ort der tatsächlichen Operation, das heißt beim Patienten, befindet, während das haptische Eingabesystem am Ort des Operateurs lokalisiert ist. Dadurch werden Notfalleingriffe oder Eingriffe durch Spezialisten ermöglicht, ohne dass diese gegebenenfalls weite Reisen mit dem damit verbundenen Stress und der Ermüdung auf sich nehmen müssen. Allgemein bietet ein solches medizinisches Instrumentensystem auch den Vorteil, dass ein haptisches Eingabesystem so eingestellt werden kann, dass ein entsprechendes Bewegungsverhältnis zwischen der Bewegung der Hand des Benutzers, das heißt des Operateurs, und der tatsächlichen Bewegung des Objekts vorliegt, so dass auch Mikroeingriffe vorgenommen werden können. Die entsprechenden Bewegungen des Operateurs werden dann beispielsweise maßstabsgetreu umgesetzt, das heißt verkleinert. Durch die Verwendung der erfindungsgemäßen Griffelemente, Adapterelemente oder Greifereingabemodule und des haptischen Eingabesystems ergibt sich so der Vorteil, dass insbesondere bei der Anwendung von Greifbewegungen durch einen Benutzer, wie sie zum Beispiel für ein tatsächliches Ergreifen mit einer Klemme, ein Schneiden oder ähnliche Bewegungen vorgenommen werden können, eine ideale haptische Rückkopplung für den Operateur, also den Benutzer des haptischen Eingabesystems, ermöglicht werden kann. Dies verbessert letztendlich auch das Gesamtergebnis der Operation und trägt somit auch zur Verbesserung der Erfolgschancen und der Genesung eines Patienten bei.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: eine schematische perspektivische Darstellung eines Greifereingabemoduls, mit Griffelement und Adapterelement, entsprechend der vorliegenden Erfindung, angeordnet an einem haptischen Eingabesystem,
- Fig. 2: eine schematische Schnittdarstellung eines Griffelements der vorliegenden Erfindung,
- Fig. 3: eine schematische Schnittdarstellung eines Adapterelements der vorliegenden Erfindung,
- Fig. 4: eine schematische Schnittdarstellung des haptischen Eingabesystems aus Fig. 1 mit einem Greifereingabemodul entsprechend der vorliegenden Erfindung,
- Fig. 5: eine schematische Schnittdarstellung eines Griffelements und Adapterelements entsprechend einem Ausführungsbeispiel der vorliegenden Erfindung,
- Fig. 6: ein weiteres Ausführungsbeispiel eines Griffelements und eines Adapterelements der vorliegenden Erfindung mit Sensoren im Adapterelement,
- Fig. 7: ein weiteres Ausführungsbeispiel von Griffelement und Adapterelement entsprechend der vorliegenden Erfindung mit Sensoren und Aktuatoren im Griffelement,
- Fig. 8: ein weiteres Ausführungsbeispiel eines Griffelements und Adapterelements entsprechend der vorliegenden Erfindung,
- Fig. 9: ein weiteres Ausführungsbeispiel eines Griffelements entsprechend der vorliegenden Erfindung mit einem Force-Feedback-System,
- Fig. 10: eine vergrößerte Schnittdarstellung der Wirkfläche des in Fig. 9 dargestellten Force-Feedback-Systems entsprechend der Linie X-X aus Fig. 9,
- Fig. 11: eine weitere beispielhafte Ausführungsform von Griffelement und Adapterelement entsprechend der vorliegenden Erfindung, und
- Fig. 12: eine schematische Darstellung eines medizinischen Instrumentensystems der vorliegenden Erfindung.

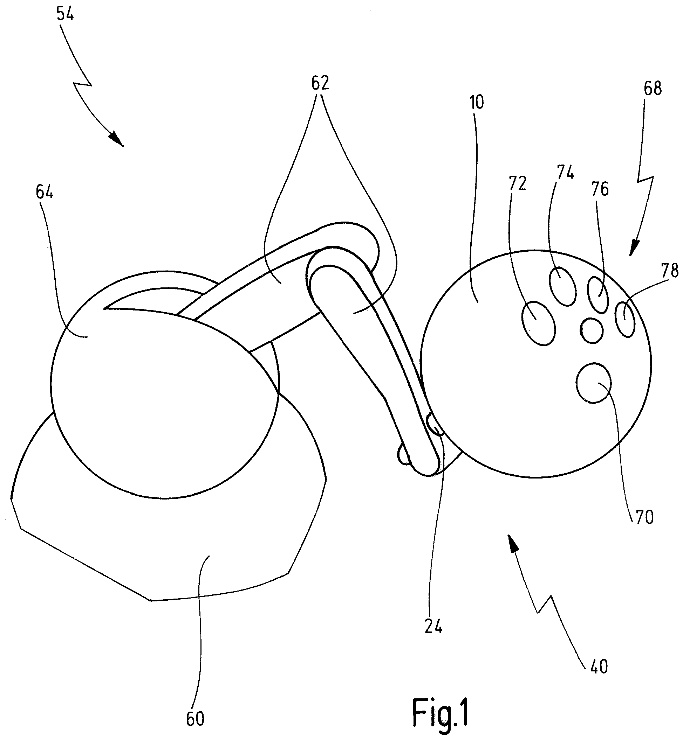

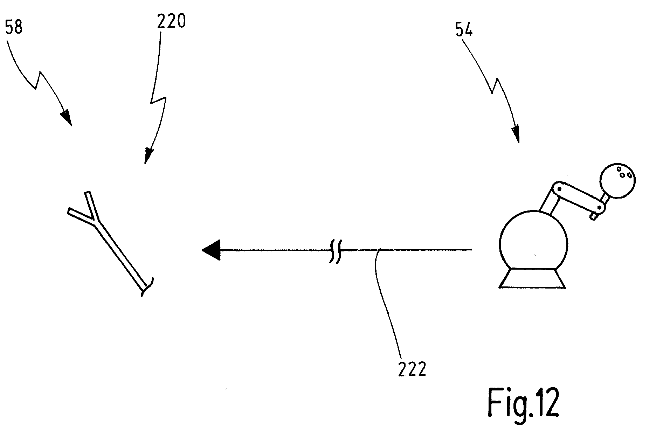

Ein erfindungsgemäßes Griffelement wird im Zusammenhang mit den Fig. 1, 2 und 4 bis 11 gezeigt und beschrieben und wird in seiner Gesamtheit jeweils mit den Bezugsziffern 10, 12, 14, 16, 18 und 20 bezeichnet. Ein erfindungsgemäßes Adapterelement wird im Zusammenhang mit den Fig. 1, 3, 4 bis 8 und 11 gezeigt und beschrieben und ist in seiner Gesamtheit jeweils mit den Bezugsziffern 24, 26, 28, 30 und 32 bezeichnet. Ein erfindungsgemäßes Greifereingabemodul wird im Zusammenhang mit den Fig. 1, 4 bis 8 und 11 näher gezeigt und beschrieben und ist jeweils in seiner Gesamtheit mit den Bezugsziffern 40, 42, 44, 46 und 48 bezeichnet. Ein erfindungsgemäßes haptisches Eingabesystem wird im Zusammenhang mit den Fig. 1, 4 und 12 gezeigt und näher beschrieben und ist in seiner Gesamtheit mit der Bezugsziffer 54 bezeichnet. Ein erfindungsgemäßes medizinisches Instrumentensystem wird im Zusammenhang mit der Fig. 12 näher gezeigt und beschrieben und ist in seiner Gesamtheit mit der Bezugsziffer 58 bezeichnet.

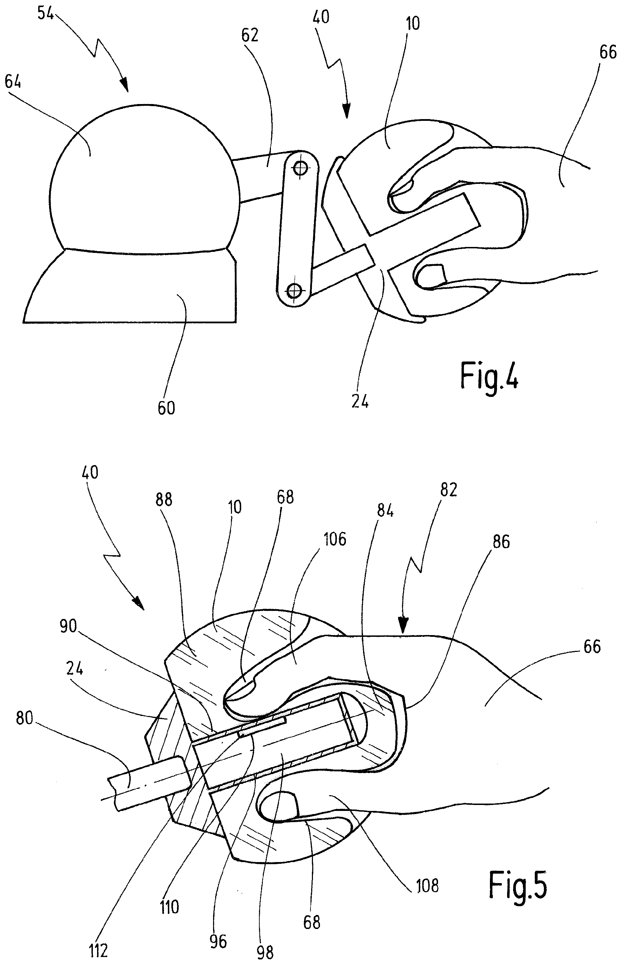

Im Zusammenhang mit den Fig. 1 bis 5 ist ein haptisches Eingabesystem 54 mit seinen einzelnen Komponenten, dem Greifereingabemodul 40 und einer Basis 60 gezeigt. An der Basis 60 ist das Greifereingabemodul 40 über eine Anordnung 62 angeordnet. Diese Anordnung 62 und die drehbare Anordnung eines Aufsatzes 64 auf der Basis 60 erlauben es, dass das Greifereingabemodul 40 in sechs Freiheitsgraden bewegt werden kann. Diese sechs Freiheitsgrade setzen sich zusammen aus drei translatorischen und drei rotatorischen Freiheitsgraden. Somit kann das Greifereingabemodul von einer Hand 66 eines Benutzers, wie beispielhaft in Fig. 4 und 5 angedeutet, an jede Position mit Bezug zu der Basis 60 bewegt werden. Mit anderen Worten folgt das Greifereingabemodul 40 entsprechend den Bewegungen der Hand 66, lediglich beschränkt durch die Ausgestaltung in Länge und Drehbarkeit der Basis 60 mit Anordnung 62 und Aufsatz 64.

Das Greifereingabemodul 40 weist das Griffelement 10, angeordnet auf dem Adapterelement 24, auf. Hierzu ist das Adapterelement 24 über die Anordnung 62 an der Basis 60 angeordnet. Diese Anordnung ist entsprechend vorzugsweise kraft-, form- und/oder stoffschlüssig, so dass eine Bewegung des Adapterelements 24 auf entsprechende Bewegungen der Anordnung 62 und des Aufsatzes 64 übertragen wird. Ebenso ist das Griffelement 10 mit dem Adapterelement 24 verbunden. Dadurch kann ein Benutzer, der über das Griffelement 10 mit dem gesamten haptischen Eingabesystem 54 in Kontakt tritt, die entsprechenden zuvor beschriebenen Bewegungen in den sechs Freiheitsgraden veranlassen.

Zum Inkontakttreten eines Benutzers (nicht gezeigt) mit dem haptischen Eingabesystem 54 weist das Griffelement 10 eine Aufnahme 68 auf. Diese Aufnahme 68 weist ihrerseits in diesem Ausführungsbeispiel fünf Löcher 70, 72, 74, 76 und 78 auf. In diese Löcher 70, 72, 74, 76, 78 können die jeweiligen Finger einer Hand 66 des Benutzers aufgenommen werden. Dadurch kann der Benutzer mit seiner Hand 66 das Griffelement 10 ergonomisch ergreifen und somit die entsprechenden zuvor beschriebenen Bewegungen durchführen. Das Griffelement 10 ist somit ergonomisch geformt. Wenngleich die Aufnahme 68 hier in einzelne Löcher 70 bis 78 unterteilt ist, ist es auch durchaus denkbar, eine entsprechende Aufnahme 68 jeweils in eine, zwei, drei oder vier Öffnungen zu unterteilen, die jeweils dann die gesamte Hand 66, oder auch nur einzelne Finger, aufnehmen. Dies kann letztendlich zu einer nahezu rotationssymmetrischen Ausgestaltung der Aufnahme 68 im Griffelement 10 mit Bezug auf eine Längsachse 80 des Adapterelements 24 führen. Dadurch wäre ein entsprechender Benutzer darin frei, wie er seine Hand 66 in die Aufnahme 68 des Griffelements 10 einführt. Um ein Verrutschen der Finger der Hand 66 zu verhindern, kann die Aufnahme 68 dann zusätzlich zumindest einzelne Mulden aufweisen, in denen die jeweiligen Finger aufgenommen sind. Insgesamt ist die Aufnahme 68 in allen Varianten so ausgestaltet, dass sie vorzugsweise an einem proximalen Ende 82 eine Verdickung 84 aufweist. Diese Verdickung 84 sorgt für eine ergonomische Aufnahme der Hand, indem die Handinnenfläche 86 auf dieser Verdickung 84 ruhen kann. In den im Folgenden dargestellten Schnittdarstellungen ist in Übereinstimmung mit den zuvor beschriebenen Ausgestaltungsvarianten die Aufnahme 68, bzw. deren Pendants in den folgenden Ausführungsbeispielen, jeweils als eine Aufnahme dargestellt. Dies schließt jedoch nicht die Unterteilung in einzelne Abschnitte, wie zum Beispiel in Fig. 1 dargestellt, aus.

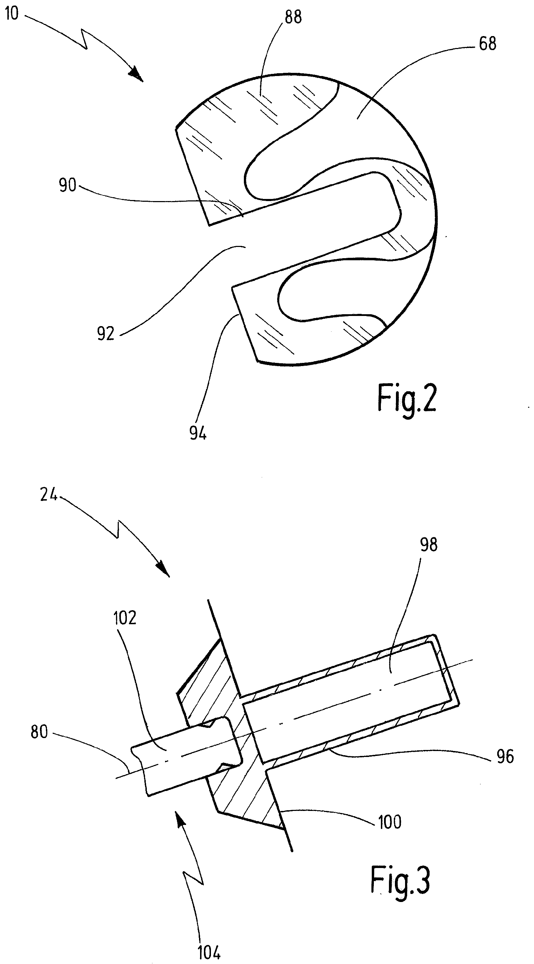

Fig. 2 zeigt eine Einzeldarstellung eines erfindungsgemäßen Griffelements 10. Dieses Griffelement 10 weist die entsprechende Aufnahme 68 sowie einen Körper 88 auf. Dieser Körper 88 ist in dem vorliegenden Ausführungsbeispiel kugelförmig ausgestaltet. Diese kugelformartige Ausgestaltung trägt ferner zur Ergonomie bei der Benutzung des Griffelements 10 durch eine Hand 66 des Benutzers bei. In dem Körper 88 des Griffelements 10 befindet sich ferner ein Verbindungselement 90 in Form einer Öffnung 92. Dieses Verbindungselement 90 dient zur kraftschlüssigen Anordnung des Griffelements 10 an das Adapterelement 24, wie dies im Folgenden noch näher beschrieben wird. Neben der Öffnung 92 weist das Verbindungselement 90 auch eine Aufliegekante 94 auf.

Fig. 3 zeigt das Adapterelement 24. Das Adapterelement 24 weist ein Aufnahmeelement 96 in Form eines stabförmigen Elements 98 und einer Aufliegefläche 100 auf. Dieses stabförmige Element 98 wird in die Öffnung 92 eines Griffelements 10 eingefügt bzw. das Griffelement 10 mit seiner Öffnung 92 auf dieses stabförmige Element 98 aufgesteckt. Nach dem kompletten Einfügen bzw. Aufsetzen des Griffelements 10 auf das Adapterelement 24 kommt die Aufliegekante 94 auf der Aufliegefläche 100 zu liegen. Damit sind Verbindungselement 90 und Aufnahmeelement 96 miteinander verbunden. Mit anderen Worten liegt damit eine Verbindung von Griffelement 10 und Adapterelement 24 vor. Diese Verbindung zwischen Griffelement 10 und Adapterelement 24 ist dann beispielsweise durch Wahl von Materialien mit einer vergleichsweise hohen Reibung untereinander kraftschlüssig. So kann das Material des Körpers 88 des Griffelements 10 beispielsweise ein elastisches Material, wie zum Beispiel Silikon, sein, welches auf einem Untergrund wie Kunststoff oder Metall für das Adapterelement 24 zu einer hohen Reibung führt. Dadurch kann verhindert werden, dass eine Drehung des Griffelements 10 um die Längsachse 80 des Adapterelements 24 auftritt. Weitere Möglichkeiten, eine solche Drehung zu verhindern, wären formschlüssige Verbindung, z.B. durch Vorsehen einer Nut- und Federanordnung oder zusätzliche Befestigungselemente wie Stifte oder dergleichen, um nur einige Beispiele zu nennen.

Das Adapterelement 24 weist ferner eine Verbindung 102 auf, über die das Adapterelement 24 und damit auch das Greifereingabemodul 40 an einem haptischen Eingabesystem 54 angeordnet werden kann. Diese Verbindung 102 dient in dem vorliegenden Beispiel als Schnittstelle 104 zu dem haptischen Eingabesystem 54. Über diese Schnittstelle 104 kann entsprechend eine Kommunikation mit dem haptischen Eingabesystem erfolgen. Hierbei meint "Kommunikation" zum einen die Möglichkeit, durch die Bewegung des Greifereingabemoduls, das heißt des Adapterelements 24, eine entsprechende Registrierung der Bewegung in dem haptischen Eingabesystem 54 zu erreichen. Ferner schließt "Kommunikation" auch den Austausch weiterer Messdaten und sonstiger Informationen mit ein, der zum Beispiel durch beliebige Kommunikationskanäle wie zum Beispiel elektrische Leitungen, Fluidleitungen, etc. ermöglicht werden kann. Solche weiteren Kommunikationen sind im Zusammenhang mit dem Beispiel des vorliegenden Greifereingabemoduls 40 nicht näher gezeigt, werden aber im Zusammenhang mit den folgenden Ausführungsbeispielen noch näher erläutert und sind ohne Weiteres auf das vorliegende Ausführungsbeispiel übertragbar.

Fig. 4 und 5 zeigen beispielhaft in den schematischen Darstellungen die Aufnahme einer Hand 66 in das Griffelement 10 eines haptischen Eingabesystems 54, bzw. eines Greifereingabemoduls 40. Die Hand 66 kommt dabei mit ihren Fingern in der Aufnahme 68 zu liegen. Für eine ergonomische Benutzung ist ferner die zuvor bereits erwähnte Auflage der Handinnenfläche 86 auf die Verdickung 84 von Vorteil. Wie insbesondere im Zusammenhang mit Fig. 5 gut zu sehen ist, verlaufen beispielhaft dargestellte Finger 106 und 108 der Hand 66 eines Benutzers so, dass sie in distaler Richtung mehr oder weniger stark aufeinander zu laufen. Mit anderen Worten ist in bevorzugten Ausgestaltungen ein jeweiliges Griffelement, hier das Griffelement 10, so ausgestaltet, dass es vom Benutzer mit seiner Hand 66 so ergriffen werden kann, dass entsprechende Finger 106, 108 in einer greifenden Haltung zueinander angeordnet sind.

Das Material des Körpers 88 des Griffelements 10 ist in dieser Ausgestaltung so angeordnet, dass sich zwischen dem Adapterelement 24 und den Fingern 106 und 108 der Hand 66 ebenfalls Material des Körpers 88 befindet. Mit anderen Worten ist jeder Finger 106, 108 von dem Adapterelement 24 durch den Körper 88 getrennt. Ferner ist in diesem Ausführungsbeispiel auch jeder Finger 106, 108 ober- und unterhalb komplett von dem Körper 88 bzw. dessen Material umgeben. Wie zuvor bereits angedeutet, ist das Material 88 im vorliegenden Ausführungsbeispiel bevorzugt aus einem elastischen Material hergestellt. Die jeweiligen Finger 106 und 108 sind somit nicht starr in der Aufnahme 68 aufgenommen, sondern können entsprechend der Flexibilität des Körpers 88 zumindest geringfügige Bewegungen durchführen. Diese Bewegungen führen mit Bezug auf die Darstellung der Fig. 5 beispielsweise nach oben bzw. nach unten. Dies entspricht mit Bezug auf das Adapterelement 24 und dessen Aufnahmeelement 96 entsprechenden Druck- und Zugbewegungen. Dabei ist unter einer Druckbewegung eine solche Bewegung zu verstehen, bei der ein Benutzer mit seiner Hand 66 im Wesentlichen eine Greifbewegung durchführt. Eine solche Greifbewegung kann unter anderem dadurch charakterisiert werden, dass entsprechende Finger, hier zum Beispiel die Finger 106 und 108, in beliebiger Weise aufeinander zu bewegt werden. Dies kann durch Bewegung von entweder dem Finger 106, dem Finger 108 oder von beiden Fingern geschehen. Eine Zugbewegung bedeutet dementsprechend eine öffnende Bewegung, die einer Greifbewegung entgegengesetzt ist. Dies bedeutet, dass eine solche öffnende Bewegung zur Folge hat, dass sich die Finger 106, 108 auseinanderbewegen, was zum Beispiel ebenfalls durch Bewegung des Fingers 106, des Fingers 108 oder durch die Bewegung beider Finger 106 und 108 erreicht werden kann. Durch die zuvor genannte Greifbewegung wird demzufolge ein Druck auf das Adapterelement 24, insbesondere auf das stabförmige Element 98 des Aufnahmeelements 96, ausgeübt. Dieser Druck wird durch das elastische Material des Körpers 88 des Griffelements 10 vermittelt.

Ist nun, wie beispielhaft in Fig. 5 angedeutet, ein Sensormittel 110 in Form eines Sensors 112 am Adapterelement 24 so angeordnet, dass es diesen entsprechenden Druck registrieren kann, führt dies zu einer Veränderung des Detektionssignals eines solchen Sensors 112. Der Sensor 112 kann hierfür zum Beispiel ein Druck- oder ein Kraftsensor sein, der die entsprechenden Änderungen des Drucks oder der ausgeübten Kraft durch den Finger 106 der Hand 66 registrieren kann. Durch die Ausgestaltung der Aufnahme 68 mit zumindest im Bereich zu einem Sensormittel 110 hin einem elastischen Material kann somit die Detektion der Bewegung bzw. die Bewegungsinformation in Form der zuvor genannten beispielhaften Greifbewegung durch das Sensormittel ermöglicht werden. Die dadurch erlangten Informationen dieser Bewegung können dann entsprechend weiter übermittelt werden und dazu benutzt werden, ein entsprechendes Objekt, das mit dem haptischen Eingabesystem 54 gesteuert werden soll, zu steuern.

Ferner ist die Ausgestaltung durch das Aufnahmeelement 96 des Adapterelements 24 so, dass es, vermittelt in diesem Beispiel durch das Griffelement 10, von den Fingern 106, 108 umfasst werden kann, so dass letztendlich aufgrund einer Wirkverbindung, die durch die Kraftübertragung von dem Finger 106 zu dem Adapterelement 24 besteht, ebenfalls diese Detektion der Bewegung bzw. der Bewegungsinformation durch das Sensormittel 110 ermöglicht wird.

Der Benutzer kann innerhalb des Griffelements 10 auch entsprechende Zugbewegungen, das heißt Öffnungsbewegungen seiner Hand 66, durchführen, die ebenfalls detektiert werden können. Hierzu kann beispielsweise eine Anordnung des Griffelements 10 an dem Adapterelement 24 über eine stoff- und/oder formschlüssige mechanische Verbindung erfolgen. Ein Beispiel einer solchen formschlüssigen mechanischen Verbindung ist zum Beispiel das Vorsehen von zumindest einer T-Nut in einem der beiden Elemente, das heißt dem Griffelement 10 oder dem Adapterelement 24, und einem entsprechenden Federelement auf dem gegenüberliegenden Element. Eine Zugbewegung würde somit ebenfalls auf den Sensor 112, also das Sensormittel 110, übertragbar sein und zum Beispiel zu einer Verminderung des Drucks oder zu einer entgegengesetzten Kraft, die detektiert werden kann, führen. Entsprechende Ausgestaltungen sind in analoger Weise auch zur Detektion von Scherkräften möglich. Eine stoffschlüssige Verbindung kann beispielsweise durch ein Verkleben der beiden Elemente, also Griffelement 10 und Adapterelement 24, erreicht werden.

Nicht beschränkende Beispiele für den Sensor 112 und auch alle im Folgenden beschriebenen Sensoren sind (resistive) Foliendrucksensoren, Piezo-Elemente, Dehnungsmessstreifen (DMS), druckabhängige oder kraftabhängige Kapazitäten, Wegencoder etc.

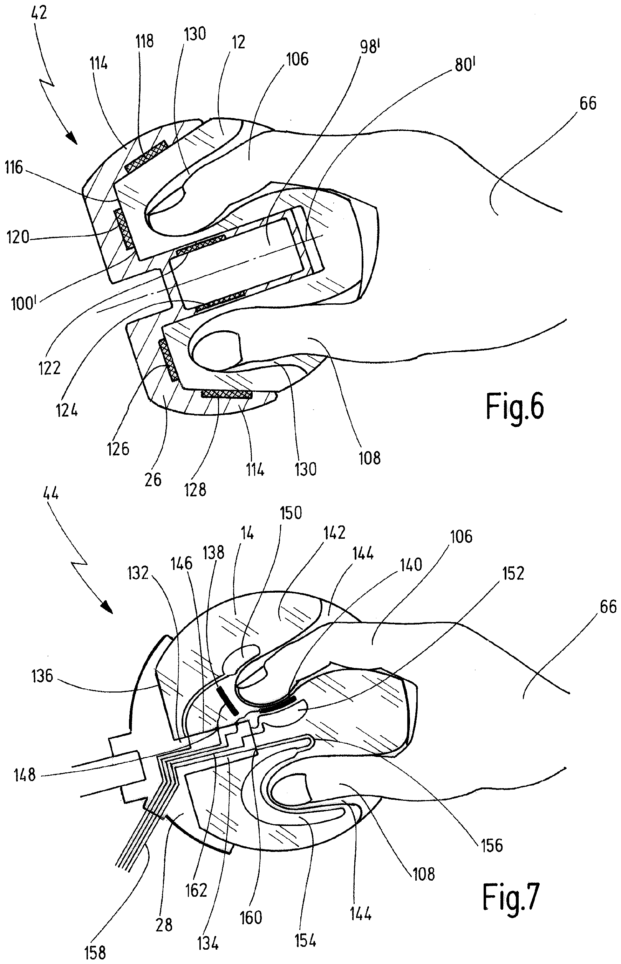

Fig. 6 zeigt eine weitere Ausgestaltung eines Greifereingabemoduls 42. Diese Greifereingabemodul 42 weist das Griffelement 12, aufgenommen an dem Adapterelement 26, auf. Das Adapterelement 26 ist im Wesentlichen wie das Adapterelement 24 ausgestaltet. Daher sind ähnliche bzw. identische Bauteile mit der gleichen Bezugsziffer, lediglich unterschieden anhand eines Striches, versehen. Im Gegensatz zum Adapterelement 24 weist das Adapterelement 26 ferner eine Seitenaufnahme 114 auf, die Bestandteil eines Aufnahmeelements 116 ist. Ferner weist das Aufnahmeelement 116 eine Aufliegefläche 100' sowie ein stabförmiges Element 98' auf. Das Griffelement 12 ist im Wesentlichen ebenfalls ausgestaltet wie das Griffelement 10 und lediglich der leicht veränderten Form bzw. Ausgestaltung des Adapterelements 26 angepasst.

Das Adapterelement 26 weist ebenfalls Sensormittel in Form von Sensoren 118, 120, 122, 124, 126 und 128 auf. Diese Sensoren sind jeweils so angeordnet, dass zum einen die Sensoren 118, 120 und 122 den Finger 106, und die Sensoren 124, 126 und 128 den Finger 108 im Wesentlichen umgeben. Da auch das Material des Griffelements 12, zumindest im Bereich zwischen dem jeweiligen Finger 106 bzw. 108 und den entsprechenden Sensoren 118 bis 128 aus einem elastischen Material besteht, führt auch hier eine Bewegung eines jeweiligen Fingers 106 bzw. 108 innerhalb einer Aufnahme 130 des Griffelements 12 dazu, dass diese Bewegung bzw. die entsprechende Bewegungsinformation durch einen solchen Sensor 118 bis 128 detektiert werden kann. Dies wird, vergleichbar zu der zuvor gemachten Ausführung, dadurch erreicht, dass durch die Elastizität des Materials des Griffelements 12 zumindest in den zuvor genannten Bereichen eine Übertragung der jeweiligen Bewegung des Fingers 106 oder 108 in Form von Druck- bzw. Kraftunterschieden oder Bewegungen auf die Sensoren 118 bis 128 erfolgt.

So kann zum Beispiel in einer beispielhaften Ausführung, wo die Sensoren 122 und 124 als Drucksensoren vorliegen, eine Greifbewegung durch die Finger 106 und 108 dazu führen, dass das entsprechende Material des Griffelements 12, das zwischen dem jeweiligen Finger 106 bzw. 108 und dem entsprechenden Sensor 122 bzw. 124 angeordnet ist, komprimiert wird, was zu einer Erhöhung des Drucks bzw. einer Weiterleitung dieses Drucks auf den jeweiligen Sensor 122 bzw. 124 führt. Der Sensor kann diese Druckänderung nun registrieren bzw. detektieren und mittels einer hier nicht näher erläuterten Elektronik des haptischen Eingabesystems in zum Beispiel eine Greifbewegung mit einer Zange, die durch das haptische Eingabesystem gesteuert werden soll, umwandeln.

Analog können die Sensoren 118 bzw. 128 eine Öffnungsbewegung detektieren, indem hier das Material des Griffelements 12 zwischen dem Finger 106 und dem Sensor 118 bzw. zwischen dem Finger 108 und dem Sensor 128 komprimiert wird, bzw. auf dieses eine Kraft ausgeübt wird. Dies kann dann durch entsprechende Druck- bzw. Kraftsensoren 118, 128 registriert werden und in Analogie zu der zuvor gemachten beispielhaften Erläuterung zu einer Öffnung einer entsprechend zu steuernden Zange führen.

Die weiteren vorgesehenen Sensoren 120 und 126 können ebenfalls zu einer Detektion dieser Greif- oder Öffnungsbewegungen, oder jeder anderen möglichen Bewegung der Finger 106 bzw. 108, beitragen und somit die Genauigkeit der Detektion erhöhen. In anderer beispielhafter Ausgestaltung können die Sensoren 120 und 126 als Bewegungssensoren ausgestaltet sein, die im Allgemeinen eine Bewegung der Finger 106, 108 detektieren können, zum Beispiel wenn sich diese aus der dargestellten Position durch Rotation verändern, was insbesondere in Bezug auf eine Längsachse 80' des Adapterelements 26 zu verstehen ist. Dies kommt insbesondere dann in Betracht, wenn es sich bei dem Griffelement 12 um ein solches Griffelement handelt, das keine Trennung der Aufnahme 130 in die jeweiligen Finger der Hand 66 aufweist, sondern eine freie Rotation der Finger der Hand 66 in der Aufnahme 130 um diese Achse 80' ermöglicht.

Allgemein kann die Sensorik somit senkrecht zu den Fingern, wie zum Beispiel die Sensoren 118 und 122 zum Finger 106, als auch tangential dazu, wie zum Beispiel der Sensor 120 zum Finger 106, angeordnet sein. Dabei sind die Angaben "senkrecht" und "tangential" hier mit Bezug auf eine Greif- oder Öffnungsbewegung der Finger 106 und 108 zu verstehen, also mit Bezug auf die Darstellung von Fig. 6 als Bewegungen im Wesentlichen nach oben und nach unten. Somit bezeichnet "senkrecht" hier die Anordnung, in der sich z.B. der Finger 106 auf die Sensoren 118 und 122 zu- bzw. von diesen wegbewegt, während "tangential" das Passieren des Sensors 120 durch den Finger 106 bei dessen Bewegung meint.

Fig. 7 zeigt eine weitere alternative Ausgestaltung in Form des Greifereingabemoduls 44. Dieses Greifereingabemodul 44 weist ein Adapterelement 28 und ein Griffelement 14 auf. Das Adapterelement 28 weist ebenfalls ein Aufnahmeelement 132 auf. Das Aufnahmeelement 132 weist seinerseits ebenfalls ein stabförmiges Element 134 sowie eine Anliegefläche 136 auf. Im Gegensatz zu den zuvor beschriebenen Ausführungsbeispielen der Adapterelemente 24 und 26 weist das Adapterelement 28 kein Sensormittel auf.

In diesem Ausführungsbeispiel des Greifereingabemoduls 44 sind hingegen Sensormittel in Form der Sensoren 138 und 140 in einem Körper 142 des Griffelements 14 vorgesehen. Diese Sensoren 138 und 140 sind analog zu der zuvor im Zusammenhang mit der Fig. 6 gemachten Beschreibung entweder senkrecht, wie der Sensor 140, oder tangential, wie der Sensor 138, zu dem Finger 106 der Hand 66 angeordnet. Ebenso, wie bei dem Greifereingabemodul 42 aus Fig. 6, kann bei dem Greifereingabemodul 44 in Fig. 7 die entsprechende Bewegung des Fingers 106 detektiert werden. Hierzu wird auch eine entsprechende Kraft bzw. ein Druck durch das Material des Körpers 142 des Griffelements 14 auf die Sensoren 140 bzw. 138 übertragen. Dies wird bevorzugterweise ebenfalls durch die Verwendung eines elastischen Materials für das Griffelement 14, zumindest im Bereich einer Aufnahme 144 des Griffelements 14, ermöglicht. Im Gegensatz zu der Ausgestaltung von dem Greifereingabemodul 42 sind vorliegend beim Greifereingabemodul 44 die Sensoren 138 und 140, wie gesagt, im Griffelement 14 angeordnet. Um eine entsprechende Wirkverbindung und damit Übertragung der Bewegungsinformationen von dem Griffelement 14 zu letztendlich dem haptischen Eingabesystem bzw. dem zu steuernden Objekt zu ermöglichen, ist vorliegend das Griffelement 14 mit einem Verbindungselement 146 versehen. Dieses Verbindungselement 146 sorgt für eine Verbindung mit dem Aufnahmeelement 132 des Adapterelements 28. Dabei führt diese Verbindung ebenfalls wieder zu einer zumindest kraftschlüssigen Anordnung des Griffelements 14 an dem Adapterelement 28. Daneben, oder auch alternativ, sind auch hier wieder form- und/oder stoffschlüssige Anordnungen denkbar. Ferner weist das Verbindungselement 146 Verbindungsstellen 148 auf, die zu einer Übermittlung der von den Sensoren 138 und 140 übermittelten Signale über das Adapterelement 28 zu einem bestimmten Zielsystem, wie zum Beispiel einem zu steuernden Objekt, dienen. An diesen Verbindungsstellen 148 wird für diese Sensoren 138 und 140 eine entsprechende Verbindung von dem Griffelement 14 zu dem Adapterelement 28, das heißt insbesondere von dem Verbindungselement 146 zu dem Aufnahmeelement 132, ermöglicht. Eine solche Verbindung kann beispielsweise durch entsprechende Metallkontakte auf beiden Seiten realisiert werden, die im Fall eines Zusammenwirkens von Griffelement 14 mit Adapterelement 28 einen gewünschten Kontakt herstellen können.

Neben den Sensoren 138 und 140 weist das Griffelement 14 ferner Aktuatoren 150, 152 und 154 auf. Diese Aktuatoren 150, 152 und 154 dienen in dem vorliegenden Ausführungsbeispiel des Griffelements 14, bzw. des Greifereingabemoduls 44, zur Übertragung einer Force-Feedback-Information, oder allgemein eines Force-Feedbacks, auf die Finger 106 und 108 der Hand 66 eines Benutzers. Hierfür sind die vorliegend gezeigten exemplarischen Aktuatoren 150, 152 und 154 als dehnbare Hohlräume, wie zum Beispiel als Ballone, ausgestaltet. In diese Hohlräume kann ein Fluid, zum Beispiel ein Gas wie Stickstoff oder Umgebungsluft, oder eine Flüssigkeit wie zum Beispiel Wasser oder ein Öl, gepumpt werden. Durch dieses Fluid wird dann das Volumen des jeweiligen Aktuators 150, 152, 154 vergrößert, bzw. beim Entfernen des Fluids verringert. Dies wird abermals durch die elastische Ausgestaltung des Körpers 142 des Griffelements 14 ermöglicht.

Eine entsprechende Vergrößerung des jeweiligen Volumens eines Aktuators 150, 152, 154 durch das Einströmen bzw. Einpumpen eines Fluides führt dann zu einer Verdrängung des Materials des Körpers 142 bzw. des Griffelements 14, das den Aktuator 150, 152, 154 umgibt. Mit Bezug auf beispielsweise den Aktuator 150 bedeutet dies, dass durch ein Expandieren des Aktuators 150 eine Verdrängung des ihn umgebenden Materials des Griffelements 14 auftritt. Diese Verdrängung des Materials wirkt sich letztendlich auf die Aufnahme 144 im Bereich des Fingers 106 aus. Dies führt in der Folge dazu, dass durch das Expandieren des Aktuators 150 ein Druck auf den Finger 106 durch das Griffelement 14 im Bereich der Aufnahme 144 auftritt. Entsprechendes ist auch auf den Aktuator 152 übertragbar. Die Aktuatoren 150 und 152 sind in dem Griffelement 14 so angeordnet, dass sie jeweils mit Bezug auch auf den Finger 106 oberhalb und unterhalb der Aufnahme 144 angeordnet sind. Dies ermöglicht somit eine Übertragung von Force-Feedback-Informationen von entweder beiden Seiten gleichzeitig, oder jeweils nur von der Ober- bzw. Unterseite mit Bezug auf den Finger 106. Hierdurch ist neben der generellen Möglichkeit, ein Force-Feedback oder eine Force-Feedback-Information an den Benutzer zu übertragen, ferner die Möglichkeit gegeben, das Force-Feedback in Abhängigkeit von dem zu steuernden Objekt zu übermitteln. Befindet sich zum Beispiel ein Widerstand oberhalb des zu steuernden Objekts, kann das Force-Feedback nur auf den Aktuator 150 zum Benutzer übertragen werden, während ein Widerstand unterhalb des Objekts oder eines Objektteils lediglich auf den Aktuatur 152 übermittelt wird und damit zu einem Force-Feedback unterhalb des Fingers 106 des Benutzers führt.

Eine alternative Ausgestaltung zeigt der Aktuator 154. Dieser ist so in dem Körper 142 des Griffelements 14 angeordnet, dass er die Aufnahme im Bereich des Fingers 108, insbesondere am distalen Ende, umgibt. Eine Betätigung dieses Aktuators 154 führt somit zu einer gleichmäßigen Übertragung des Force-Feedbacks auf den Finger 108. Die Übertragung durch einen solchen Aktuator 154 kann dadurch deutlicher und somit auch eindeutiger sein.

Um eine entsprechende Betätigung bzw. Operation der Aktuatoren 150, 152 und 154 zu erreichen, weisen diese Kanäle auf, wie beispielhaft durch den Kanal 156 für den Aktuator 154 dargestellt. Durch diese Kanäle kann ein entsprechendes Fluid in den oder aus dem Aktuator 154 (bzw. 150 und 152) strömen. Das entsprechende Fluid wird dabei in das Griffelement 14 durch einen Kanal 158 im Adapterelement 28 übertragen. Um auch hier eine Wirkverbindung zwischen dem Griffelement 14 und dem Adapterelement 28, das heißt insbesondere zwischen dem Aufnahmeelement 132 und dem Verbindungselement 146, zu ermöglichen, findet sich auch hier zwischen diesen beiden, das heißt zwischen Verbindungselement 146 und Aufnahmeelement 132, eine Verbindungsstelle 160 an beiden Elementen. Durch diese Verbindungsstelle 160 kann das Fluid von dem Kanal 158 in den Kanal 156 und somit in den Aktuator 154 strömen, bzw. aus dem Aktuator 154 über den Kanal 156 in den Kanal 158 strömen. Die Verbindungsstelle 160 kann dazu unterschiedlich ausgestaltet sein, zum Beispiel in Form von zwei sich überlagernden Öffnungen, oder aber auch in Form von einem Schlauchstecker, zum Beispiel im Bereich des Griffelements 14, der in eine Schlauchbuchse des Adapterelements 28 eingreift, oder umgekehrt. Sollen solche Aktuatoren, die durch Expansion funktionieren, in einem entsprechenden Adapterelement vorgesehen werden (nicht gezeigt), ist das Adapterelement zumindest teilweise auch vorzugsweise elastisch ausgestaltet.

In den gezeigten Aktuatoren in Form der Hohlräume für die Aktuatoren 150 bis 154 kann auch vorgesehen sein, dass die entsprechenden Aktuatoren als (kleine) elektrische Antriebe, zum Beispiel Vibrationselemente, elektroaktive Polymere, oder andere steuerbare Elemente, die letztendlich zu einer Formveränderung und damit zu einer Wirkung auf einen jeweiligen Finger 106, 108 eines Benutzers führen, ausgestaltet sind.

Neben der hier gezeigten Ausgestaltung, in der zwischen dem Verbindungselement 146 und dem Aufnahmeelement 132 eine derartige Wirkverbindung hergestellt wird, dass die Kommunikation zu Sensoren 138 und 140 bzw. von diesen, und zu bzw. von den Aktuatoren 150, 152 und 154 ermöglicht wird, kann auch vorgesehen sein, dass die entsprechenden Verbindungen für zumindest ein oder mehrere der zuvor beschriebenen Elemente, das heißt Sensoren oder Aktuatoren, direkt an dem Griffelement 14 zu einem Element des haptischen Eingabesystems oder dem zu steuernden Objekt geleitet werden, bzw. von diesem in das Griffelement 14 führen. Ferner ist neben der hier gezeigten Ausgestaltung, in der die gezeigten Leitungen, wie zum Beispiel der Kanal 158, aus dem Adapterelement 28 herausgehen, auch denkbar, dass diese intern über einen Anschluss bzw. eine Schnittstelle des Adapterelements 28 an ein haptisches Eingabesystem direkt über eingebaute Leitungen bzw. Verbindungen weitergeführt werden.

In einer weiteren, hier nicht separat noch einmal gezeigten Ausgestaltung kann ein jeweiliges Force-Feedback-Element auch als ein passives Force-Feedback-Element ausgestaltet sein. Als Beispiel sei hier auf den Aktuator 152 verwiesen, der zum Beispiel auch mit einer elektrorheologischen (oder auch magnetorheologischen) Flüssigkeit gefüllt sein kann. Diese Flüssigkeit kann dann einem elektrischen Feld ausgesetzt werden. Dies kann zum Beispiel durch eine Zuleitung 162 in dem Adapterelement 28 realisiert sein. Durch Applikation eines elektrischen Feldes auf diese elektrorheologische Flüssigkeit in dem Aktuator 152 verändert sich deren Elastizität bzw. deren Widerstand gegen eine Stauchung. Dies kann zum Beispiel dann vorgenommen werden, wenn ein zu steuerndes Objekt bei beispielsweise einer Greifbewegung auf ein eher festes Material trifft. Der Benutzer spürt dann an seiner Hand 66 über den Finger 106 ebenfalls diesen festeren Widerstand in Form der weniger elastisch gemachten elektrorheologischen Flüssigkeit des Aktuators 152.

Umgekehrt kann durch Anpassung des elektrischen Feldes über die Zuleitung 162 die elektrorheologische Flüssigkeit im Aktuator 152 elastischer bzw. mit einem geringeren Widerstand gegen Stauchung versehen werden. Dies wäre zum Beispiel dann angebracht, wenn ein entsprechendes zu steuerndes Objekt auf ein eher weicheres Material trifft. Der Benutzer würde dies hier ebenfalls als haptische Rückkopplung, das heißt als eine Art Force-Feedback, über seinen Finger 106 erfahren. Da diese Rückkopplung bzw. dieses Feedback mit diesen elektrorheologischen Flüssigkeiten nicht zu einer direkten Auswirkung auf den ruhenden Finger 106 führt, wie dies zum Beispiel bei den zuvor beschriebenen aktiven Force-Feedback-Varianten mit elektroaktiven Polymeren, fluidgefüllten Hohlräumen etc., der Fall ist, wird hier im Rahmen der vorliegenden Erfindung von passivem Force-Feedback gesprochen. Neben der genannten Variante mit elektrorheologischen Flüssigkeiten sind entsprechend auch magnetorheologische Flüssigkeiten oder andere Materialien, die kontrolliert ihre elastischen Eigenschaften verändern können, möglich. Bei magnetorheologischen Flüssigkeiten erfolgen die genannten Änderungen aufgrund eines veränderbaren magnetischen Feldes. Dies kann über induktive Effekte ebenfalls elektrisch gesteuert werden.

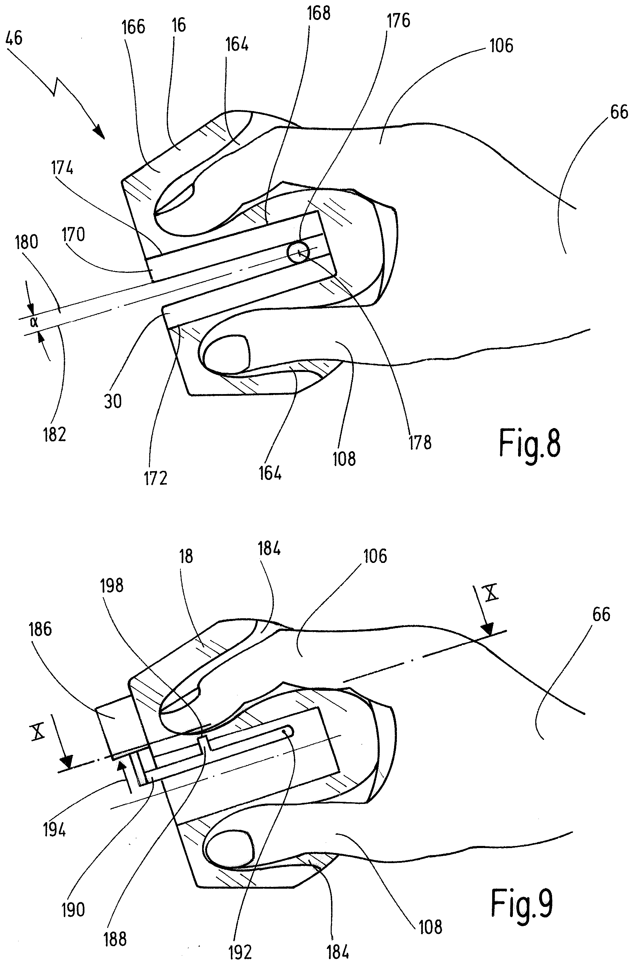

Fig. 8 zeigt die vereinfachte Darstellung eines weiteren Ausführungsbeispiels eines Greifereingabemoduls 46 der vorliegenden Erfindung. Dieses Greifereingabemodul 46 besteht ebenfalls aus einem Griffelement 16 und einem, hier vereinfacht dargestellten, Adapterelement 30. Das Griffelement 16 weist ebenfalls eine Aufnahme 164 auf, in der zumindest die Finger 106 und 108 der Hand 66 eines Benutzers aufgenommen werden können. Wie in den zuvor gezeigten Beispielen ist diese Aufnahme 164 abermals in einem Körper 166 des Griffelements 16 angeordnet. Dieser Körper 166 besteht auch im vorliegenden Beispiel des Greifereingabemoduls 46 aus einem elastischen Material. Das Griffelement 16 weist ferner ein Verbindungselement 168 auf, über das das Griffelement 16 mit dem Adapterelement 30 verbunden ist. Das Adapterelement 30 weist seinerseits zwei Paddel 170 und 172 auf. Die Seiten der Paddel 170 und 172, die den Fingern 106 und 108 zugewandt sind, treten mit dem Griffelement 16, bzw. dem Verbindungselement 168, in Kontakt und bilden damit das Aufnahmeelement 174 des Adapterelements 30. Die Paddel 170 und 172 sind zumindest über eine Verbindungsachse 176 miteinander verbunden. Diese Verbindungsachse 176 ist so in dem Adapterelement 30 angeordnet, dass sie die Paddel 170 und 172 scharnierartig miteinander verbindet, wobei sie um eine Mittelachse 178 der Verbindungsachse 176 zueinander verschwenken können. Diese Verschwenkung um die Achse 178 führt zu einer Winkeleinstellung 180 entsprechend dem Winkel α. Dieser Winkel α gibt den Winkel eines entsprechenden Paddels 170 bzw. 172 zu einer entsprechenden Längsachse 182, die den Längsachsen durch die Adapterelemente der vorherigen Ausführungsbeispiele vergleichbar ist, an. Der Winkel zwischen den Paddeln 170 und 172 errechnet sich somit zu 2 x a. Die Verbindungsachse 176 ist bevorzugterweise so ausgestaltet, dass sie eine von außen verursachte Veränderung des Winkels 180 der Paddel 170 und 172 detektieren kann. Hierzu weist die Verbindungsachse 176 vorzugsweise einen Winkelencoder (nicht gezeigt) auf.

Durch diese Variante ist somit nun ebenfalls ein Sensormittel in Form der Verbindungsachse 176, die ebenfalls eine Detektion der Bewegung der Paddel 170 und 172 vornehmen kann, gegeben. Diese Detektion geschieht hier im Wesentlichen dadurch, dass, veranlasst durch eine Greifbewegung mit den Fingern 106 und 108 und ermöglicht durch die elastische Ausgestaltung des Körpers 166 des Griffelements 16, ein Zusammenbewegen der Paddel 170 und 172 um die Achse 178 erfolgt. Mit anderen Worten wird der Winkel α zwischen einem jeweiligen Paddel 170 bzw. 172 und der Längsachse 182 verringert. In gleicher Weise kann auch eine Öffnungsbewegung, bei der sich die Finger 106 und 108 auseinanderbewegen, dazu führen, dass sich die Paddel 170 und 172 auseinanderbewegen. Das Resultat ist eine Vergrößerung des jeweiligen Winkels 180 bzw. a, was ebenfalls durch die Verbindungsachse 176 detektiert werden kann. Um sowohl Zug- als auch Druckbewegungen durch die Finger 106 und 108 mit der Verbindungsachse 176 detektieren zu können, ist das Griffelement 16 mit dem Adapterelement 30, das heißt insbesondere das Verbindungselement 168 mit dem Aufnahmeelement 174, kraft- und vorzugsweise auch form- und/oder stoffschlüssig verbunden. Dies kann zum Beispiel durch das Vorsehen von T-Nuten und entsprechenden Federn, wie zuvor bereits beschrieben, realisiert werden. Daneben sind auch Ausgestaltungen denkbar, wo entsprechende feste Verbindungen durch Stifte, Schrauben, Nieten oder dergleichen vorgesehen werden. Außerdem kann die entsprechende Verbindung auch durch eine Klebeverbindung zwischen dem Aufnahmeelement 174 und dem Verbindungselement 168 realisiert werden.

Durch die so durchgeführte Greifbewegung und das entsprechende Nachgeben des Griffelements 16 bzw. des Greifereingabemoduls 46, ist diese Ausgestaltung des Greifereingabemoduls 46 besonders ergonomisch und hinsichtlich des Bedienens für einen Benutzer sehr komfortabel.

Ferner weist das Greifereingabemodul 46 in einer weiteren bevorzugten Ausgestaltung einen Motor (nicht gezeigt) auf. Dieser Motor steht mit der Verbindungsachse 176 in Wirkverbindung und ist beispielsweise an oder in dieser angeordnet. Durch diese Anordnung kann der Motor die Verschwenkung der Paddel 170 und 172 um die Achse 178 steuern. Mit anderen Worten ist es somit möglich, die Paddel 170 und 172 über den Motor zu betätigen und im Winkel zueinander zu verändern. Durch die Betätigung des Motors weist das Greifereingabemodul 46 somit auch die Möglichkeit auf, eine Force-Feedback-Information bzw. allgemein ein Force-Feedback an einen Benutzer zurückzugeben. Hierzu kann im vorliegenden Beispiel neben einem aktiven Drücken auch ein aktives Zusammenziehen der Finger 106 und 108 erfolgen. Somit können alle Wirkungen, die auf ein zu steuerndes Objekt, wie zum Beispiel eine Klemme, Zange oder Schere, wirken können, auch auf dieses Greifereingabemodul 46 übertragen werden. Das Force-Feedback kann hierbei sowohl die Form eines öffnenden oder schließenden Drucks, wie zuvor erwähnt, als auch die Übertragung in Form von Vibrationen aufweisen.

Neben diesen aktiven Force-Feedback-Varianten durch den Motor kann dieser auch Eigenschaften eines zuvor bereits erwähnten passiven Force-Feedbacks aufweisen. Hierzu kann der Motor über eine Betätigung an der Verbindungsachse 176 einer entsprechenden Bewegung durch die Finger 106 und 108 des Benutzers entgegen- bzw. mitwirken. Der Benutzer bekommt somit an seiner Hand 66, bzw. den Fingern 106 und 108, den haptischen Eindruck, als würde er beispielsweise auf ein sehr hartes oder sehr weiches Objekt mit dem zu steuernden Objekt treffen. In einem expliziten Beispiel kann dabei ein Schnitt mit einem zu steuernden Instrument durch weiches Gewebe begünstigt werden, während beim Auftreffen auf Knorpel oder Knochengewebe der Widerstand an dem Greifereingabemodul 46 erhöht wird. Dies geschieht entsprechend durch die entgegengesetzte oder begünstigende Betätigung des Motors in Bezug auf eine entsprechende Greifbewegung.

Fig. 9 zeigt eine weitere beispielhafte Ausgestaltung eines Force-Feedback-Systems anhand des Griffelements 18. Hierbei weist das Griffelement 18 ebenfalls eine Aufnahme 184 auf, in der zumindest die Finger 106 und 108 der Hand 66 des Benutzers aufgenommen sind. An dem Griffelement 18 ist ferner ein Aktor 186 angeordnet, über den ein Tastelement 188 betätigt werden kann. Hierzu ist eine kraft- bzw. vorzugsweise formschlüssige Verbindung über ein Gestänge 190 vorgesehen. Das Tastelement 188 ist so in dem Griffelement 18 angeordnet, dass dies durch kleine Öffnungen, wie im Folgenden noch näher beschrieben wird, direkt durch das Griffelement 18 in die Aufnahme 184 eintreten und somit mit dem Finger 106 des Benutzers in Berührung kommen kann.

Der Aktor 186 kann dann über das Gestänge 190, das an einer Achse 192 aufgehängt ist, das Tastelement 188 in Richtung auf den Finger 106 zu, das heißt mit Bezug auf die Darstellung von Fig. 9 nach oben, bewegen. Dies ist durch einen Pfeil 194 angedeutet. Diese Bewegung nach oben entsprechend dem Pfeil 194 kann als eine kontinuierliche, dauerhaft drückende Bewegung gegen den Finger 106 ausgeführt werden. Daneben ist bevorzugterweise eine Ausgestaltung möglich, in der das Tastelement 188 gegen den Finger 106 vor- und zurückbewegt wird, so dass eine Vibration durch das Tastelement 188 am Finger 106 entsteht. Der Aktor 186 und die Ausgestaltung mit Gestänge 190 kann dabei dann so sein, dass eine entsprechende Vor- und Zurückbewegung, das heißt entsprechend dem Pfeil 194 und entgegengesetzt, aktiv möglich ist. Daneben ist auch denkbar, dass nur eine Bewegungsrichtung entsprechend dem Pfeil 194 (oder umgekehrt) möglich ist, während die entgegengesetzte Bewegungsrichtung sich aufgrund einer Vorspannung, zum Beispiel mittels einer Feder, ergibt.

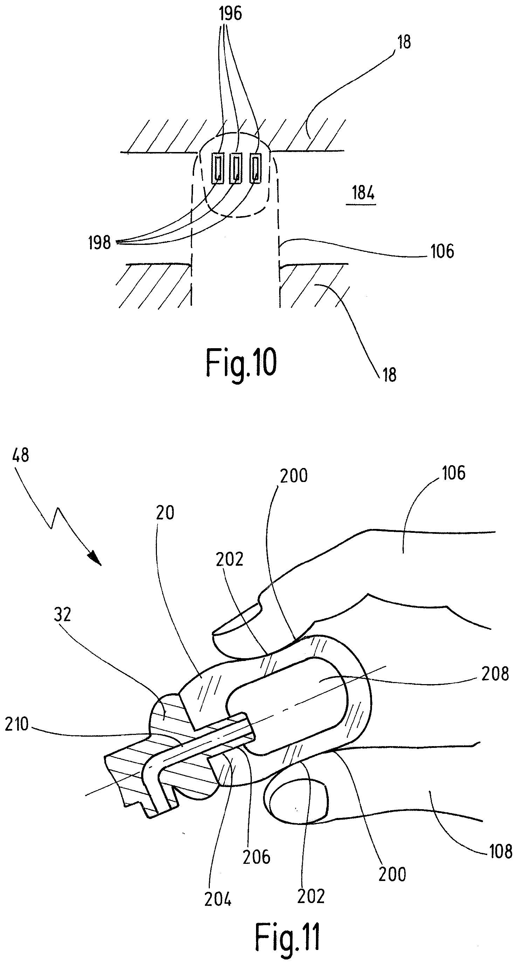

Fig. 10 zeigt hierbei entsprechend eine Schnittansicht, wobei auf das Tastelement 188 draufgeblickt wird. Fig. 10 zeigt somit das Griffelement 18 und den Finger 106, letzterer ist der Übersichtlichkeit halber gestrichelt dargestellt. Unterhalb des Fingers 106 befinden sich Öffnungen 196 im Griffelement 18 bzw. der Aufnahme 184. Durch diese Öffnungen 196 können Lamellen 198, die an dem Tastelement 188 proximal angeordnet sind, hindurchtreten. Diese Ausgestaltung ist insbesondere daher von Vorteil, da ein Finger bzw. dessen Tastsinn sehr feinfühlig und empfindlich auf Vibrationen reagiert. Dies kann beispielsweise durch die hier gezeigte Anordnung mit den Lamellen 198, aber auch durch andere Strukturen, wie zum Beispiel ein Punktraster oder auch eine flächenartige Ausgestaltung, umgesetzt werden.

Neben einer Ausgestaltung mit Öffnungen 196 ist es ferner denkbar, hier lediglich eine Materialschwächung zu haben, so dass eine Vibration durch die Lamellen 198 den Finger 106 erreichen kann, die Aufnahme 184 im Übrigen aber geschlossen ist.

In den zuvor gezeigten Ausführungsbeispielen der Griffelemente 12 bis 18 sind diese vorzugsweise elastisch ausgestaltet. Hierbei kommen insbesondere elastische Polymere, wie zum Beispiel Silikon, das vorzugsweise für medizinische Zwecke geeignet ist, in Betracht. Daneben liegen grundsätzlich aber auch Ausgestaltungen mit starren oder zumindest teilweise starren Körpern der jeweiligen Griffelemente im Rahmen dieser Erfindung. Die Griffelemente sind vorzugsweise ferner durch eine selbsttragende Form gekennzeichnet, in die eine Hand 66 des Benutzers aufgenommen werden kann.

Wie bereits zuvor beispielhaft erwähnt, kann ein entsprechendes Griffelement als ein auswechselbarer Überzug bzw. ein auswechselbares Element ausgestaltet sein. Dabei ist dann selbstverständlich bevorzugt, dass der Wert und der Materialaufwand sowie der Herstellungsaufwand, der mit dem jeweiligen Griffelement verbunden ist, möglichst geringgehalten werden, um hier ein möglichst einfach handhabbares und unempfindliches Einweg- bzw. Mehrwegprodukt bereitzustellen.

Neben den gezeigten Ausführungsbeispielen, bei denen jeweils ein Sensor in einer Richtung den jeweiligen Fingern 106 und 108 zur Verfügung steht, ist es im Rahmen der vorliegenden Erfindung selbstverständlich auch denkbar, eine Anordnung mehrerer Sensoren nebeneinander, zum Beispiel in Form von Sensorarrays, zu ermöglichen. Dadurch kann beispielsweise jedem Finger einer Hand 66 ein entsprechender Sensor zugeordnet werden und somit die Bewegung jedes Fingers separat erfasst werden. Auf diese Weise können dann entweder auch einzelne Objektelemente von einem zu steuernden Objekt durch die Finger einzeln betätigt werden, oder unterschiedlichen Fingern unterschiedliche Funktionen für ein zu steuerndes Objekt zugeordnet werden. Um diese Anordnung zu begünstigen, kann ein entsprechendes Griffelement vorzugsweise mit entsprechenden Unterbrechungen oder sonstigen Materialänderungen zwischen den entsprechenden Fingern in einer jeweiligen Aufnahme versehen sein. Dadurch können Druckverteilungen, die durch eine Betätigung mittels eines Fingers in dem Material eines entsprechenden Griffelements entstehen, unterbunden werden.

Alternativ kann ein entsprechendes Griffelement mit einem gemeinsamen Sensorelement zur Erfassung der Bewegung eines der vorhandenen Finger vorgesehen sein. So kann ermöglicht werden, dass unabhängig von der genauen Position der Finger in einer entsprechenden Aufnahme eines Griffelements, das heißt zum Beispiel der rotatorischen Anordnung mit Bezug auf eine Längsachse, immer eine verlässliche Betätigung des fernen und zu steuernden Objekts ermöglicht wird. Dies kann zum Beispiel dadurch realisiert werden, dass ein Sensor von einem Material im Griffelement umgeben ist, das für eine entsprechende Kräfteverteilung sorgt. So kann dann beispielsweise durch die Betätigung mit dem kleinen Finger genauso wie durch Betätigung mit dem Zeigefinger ein gleicher Effekt am zu steuernden Objekt hervorgerufen werden, indem ein gemeinsamer Sensor dies aufgrund der Kräfteverteilung gleich detektiert.

Diese zuvor genannten Ausgestaltungen mit Sensorarrays, Druck- bzw. Kräfteverteilung oder Begrenzung dieser Druck- bzw. Kräfteverteilung ist in analoger Weise auch auf die Aktuatoren zur aktiven, aber auch passiven Force-Feedback-Übertragung möglich.

Die Griffelemente 10 bis 18 stellen ferner dahingehend eine bevorzugte Ausgestaltung der Erfindung dar, als sie die jeweiligen Finger einer Hand zumindest in zwei Richtungen, vorzugsweise ober- und unterhalb, umschließen. Dies ermöglicht neben einer verlässlichen Bedienung des haptischen Eingabesystems und einer besseren Ergonomie auch die Integration der Erkennung von Zugbewegungen, das heißt Öffnungsbewegungen der Finger.

In Fig. 11 ist ein weiteres Ausführungsbeispiel eines Greifereingabemoduls 48 gezeigt. Dieses Greifereingabemodul 48 weist ein Griffelement 20 und ein Adapterelement 32 auf. Das Adapterelement 32 weist seinerseits ein Aufnahmeelement 204 auf, das das Griffelement 20 über ein Verbindungselement 206 von diesem kraftschlüssig aufnimmt. Das Griffelement 20 weist seinerseits eine Aufnahme 200 zur Aufnahme von Fingern 106 und 108 eines Benutzers auf. Diese Aufnahme 200 ist, im Gegensatz zu den vorherigen Aufnahmen der zuvor gezeigten Griffelemente 10 bis 18, nicht an der Außenseite der Finger 106 und 108 geschlossen. Zur festen Aufnahme der Finger 106 und 108 weist dieses Griffelement 200 eine umfängliche Mulde 202 auf.

Im Inneren des Griffelements 20 ist ferner ein Hohlraum 208 vorgesehen. Dieser Hohlraum 208 kann mit einem Fluid, wie zum Beispiel einem Gas oder einer Flüssigkeit, gefüllt werden, um das entsprechende Volumen des Hohlraums 208 zu expandieren. In gleicher Weise kann ein entsprechendes Fluid aus diesem Hohlraum 208 entfernt werden, um das Volumen in dem Hohlraum 208 zu komprimieren. Dieser Austausch von Fluiden kann über das Adapterelement 32 geschehen. Hierzu ist in dem Adapterelement 32 ein Kanal 210 vorgesehen. Dieser Kanal 210 ist mit einer äußeren Einheit verbunden (nicht näher dargestellt), die die entsprechende Fluidbewegung herbeiführen kann. Hierbei kann es sich beispielsweise um einen nicht näher gezeigten Kompressor oder eine Pumpe handeln.

Führt ein Benutzer nun eine Komprimierung des Griffelements 20 durch eine Greifbewegung mit den Fingern 106 und 108 durch, führt dies letztendlich zu einer Druckerhöhung in dem Hohlraum 208, die sich über den Kanal 210 weiter auswirkt. Ein hier nicht näher gezeigtes Sensorsystem am Kanal 210 kann dann diese Druckänderung detektieren. Anhand der detektierten Druckänderungsdaten kann dann auf die entsprechende Druckkraft durch die Finger 106 und 108 zurückgeschlossen werden, in der Folge kann eine darauf basierende Betätigung eines zu steuernden Objekts, wie zum Beispieldas Schließen einer Schere oder einer Klemme, vorgenommen werden.

Aufgrund der zuvor beschriebenen Ausgestaltung des Fluidtransports durch den Kanal 210 in den Hohlraum 208 kann hierüber auch ein Force-Feedback an den Benutzer, insbesondere an seine Finger 106 und 108, ermöglicht werden. Hierzu wird zum Beispiel, wenn ein zu steuerndes Objekt auf einen Widerstand trifft, der Druck bzw. das Volumen in dem Hohlraum 208 durch Einbringen von weiterem Fluid über den Kanal 210 erhöht. Der Benutzer erhält somit ein Force-Feedback und damit die Information, dass ein entsprechend festeres Material oder ein sonstiger Widerstand für das zu steuernde Objekt vorliegt. Entgegengesetzt kann das Volumen bzw. der Druck im Hohlraum 208 über den Kanal 210 reduziert werden, indem Fluid auf diesem Wege aus dem Hohlraum 208 entfernt wird. Der Benutzer erhält somit dann ein Force-Feedback, dass kein Widerstand oder ein weicheres Material im Bereich des zu steuernden Objekts vorliegt.

Dieses Force-Feedback kann entweder als passives Force-Feedback, durch bloße Einstellung des entsprechenden Drucks bzw. Volumens im Hohlraum 208, vorgenommen werden. Daneben ist jedoch auch die Ausgestaltung als aktives Force-Feedback möglich, das beispielsweise auch als Vibration mit schnell aufeinander schwankenden Kompressions- und Dekompressionsvorgängen des Hohlraums 208 ein aktives Force-Feedback an den Benutzer über dessen Finger 106 und 108 übermittelt.

Da in dem hier gezeigten Beispiel sowohl die Detektion des vom Benutzer ausgeübten Drucks, als auch des Drucks, welcher durch die Erhöhung des Fludgehalts in dem Hohlraum 208 entsteht, über einen Kanal 210 realisiert werden soll, ist hierbei vorzugsweise eine entsprechende Rückkopplung zwischen dem Detektionssystem und dem Fluidversorgungssystem vorzusehen. Damit werden Lesefehler in dem Detektionssystem, zum Beispiel über einen Drucksensor, vermieden, die sonst dadurch auftreten könnten, dass eine Erhöhung des Drucks, mit dem Ziel, ein Force-Feedback zu liefern, letztendlich dazu führt, dass das System anhand des Drucksensors eine vermeintliche Bewegung der Finger 106 und 108 detektiert.

Entsprechend der zuvor gemachten Ausführungen ist auch das Material des Griffelements 20 als elastisches Material ausgeführt. So kann auf einfache Weise die Übermittlung der Druckinformation von den Fingern zu dem Hohlraum 208 bzw. von dem Hohlraum 208 zu den Fingern 106 und 108 erfolgen.

Fig. 12 zeigt abschließend schematisch die Ausgestaltung des medizinischen Instrumentensystems 58. Dieses weist ein zu steuerndes Objekt 220 in Form eines hier schematisch dargestellten Schneidwerkzeugs auf. Demgegenüber weist das medizinische Instrumentensystem ferner ein haptisches Eingabesystem, hier beispielsweise das haptische Eingabesystem 54, auf. Das haptische Eingabesystem 54 steht mit dem zu steuernden Objekt 220 in Wirkverbindung. Diese Wirkverbindung ist hier beispielhaft durch einen Pfeil 222 angedeutet. Aufgrund dieser Wirkverbindung kann das zu steuernde Objekt 220 basierend auf Bewegungen und Operationen am haptischen Eingabesystem 54 gesteuert werden. Neben einer Steuerung des zu steuernden Objekts 220 in den bereits zuvor erwähnten sechs Freiheitsgraden, das heißt in den drei Raumrichtungen und um drei zueinander orthogonale Rotationsachsen, kann das haptische Eingabesystem 54 ein entsprechend der zuvor gemachten Ausführungen ausgestaltetes Greifereingabemodul aufweisen. Dadurch wird ein Betätigen des zu steuernden Objekts 220 ermöglicht. Dies kann in dem hier gezeigten Beispiel des zu steuernden Objekts 220 in Form eines Schneidwerkzeugs zum Beispiel ein Schneidvorgang sein. Dieser Schneidvorgang kann durch eine Greifbewegung der Finger 106 und 108 in dem Greifereingabemodul, das heißt insbesondere dem jeweiligen Griffelement, ausgelöst werden. Umgekehrt kann eine Öffnungsbewegung zu einer Öffnung des zu steuernden Objekts 220 in Form des hier gezeigten Schneidwerkzeugs führen.

Die hier beschriebene Wirkverbindung 222 kann entweder durch elektrische Signale oder aber auch durch elektromagnetische Signale erfolgen. Dies schließt somit neben üblichen Verbindungen durch Kabel oder sonstige feste Verbindungen auch kabellose Übertragungen mit ein. Hierzu können zu steuerndes Objekt 220 und haptisches Eingabesystem 54 in einem Raum oder einem Gebäude sein. Ferner ist auch eine Anordnung denkbar, bei der die Wirkverbindung 222 über größere Entfernungen möglich ist.

Bei der Anwendung des haptischen Eingabesystems 54 am selben Ort wie dem zu steuernden Objekt 220 sollte im beispielhaften Falle der Verwendung in einem chirurgischen Eingriff die Sterilität und Hygiene des haptischen Eingabesystems 54 beachtet werden. Hierzu kann das Gerät selber beispielsweise mit einer Schutzhülle versehen werden, während ein entsprechendes Griffelement, welches direkt mit dem Operateur über dessen Hand 66 in Kontakt gerät, entsprechend der vorliegenden Erfindung als einfach sterilisierbares oder Einwegprodukt ausgestaltet sein kann.

Mit Bezug auf die Darstellung von Fig. 5 betrifft die Erfindung ein Griffelement 10 zur Anordnung an einem Adapterelement 24, um ein Greifereingabemodul 40 für ein haptisches Eingabesystem zur Steuerung von zumindest einem Objekt zu bilden, mit zumindest einer Aufnahme 68 zur Aufnahme von zumindest zwei Fingern 106, 108 eines Benutzers darin, wobei die Aufnahme zumindest in einem Abschnitt in Wirkverbindung mit zumindest einem Sensormittel 110 steht, und zumindest einem Verbindungselement 90, zur Anordnung des Griffelements 10 an dem Adapterelement 24, wobei die Aufnahme 68 so ausgestaltet ist, dass eine Bewegungsinformation einer Bewegung von zumindest einem Finger 106, 108 des Benutzers in der Aufnahme 68 durch das Sensormittel 110 detektierbar und damit die Bewegungsinformation zur Steuerung des zumindest einen Objekts übertragbar ist. Die Erfindung betrifft ferner ein entsprechendes Adapterelement 24, ein aus Griffelement 10 und Adapterelement 24 bestehendes Greifereingabemodul 40 sowie ein haptisches Eingabesystem und medizinisches Instrumentensystem.

## Patentansprüche

1. Griffelement zur Anordnung an einem Adapterelement, um ein Greifereingabemodul für ein haptisches Eingabesystem zur Steuerung von zumindest einem Objekt zu bilden, das Griffelement mit zumindest einem Verbindungselement (90, 146, 168, 206) zur Anordnung des Griffelements an dem Adapterelement (24, 26, 28, 30, 32),
aufweisend zumindest eine Aufnahme (68, 130, 144, 164, 184, 200) zur Aufnahme von zumindest zwei Fingern (106, 108) eines Benutzers darin, wobei die Aufnahme zumindest in einem Abschnitt in Wirkverbindung mit zumindest einem Sensormittel (110, 112, 118, 120, 122, 124, 126, 128) steht,
wobei die zumindest eine Aufnahme (68, 130, 144, 164, 184, 200) so ausgestaltet ist, dass eine Bewegungsinformation einer Bewegung von zumindest einem Finger (106, 108) des Benutzers in der Aufnahme (68, 130, 144, 164, 184, 200) durch das Sensormittel (110, 112, 118, 120, 122, 124, 126, 128) detektierbar und damit die Bewegungsinformationen zur Steuerung des zumindest einen Objekts übertragbar ist,
wobei das Griffelement (10) einen kugelförmigen Körper (88) aufweist, wobei eine kugelförmige Oberfläche des Körpers (88) mindestens eine Öffnung für die zumindest eine Aufnahme (68, 130, 144, 164, 184, 200) aufweist,
wobei der kugelförmige Körper (88) das Verbindungselement (90) in Form einer Ausnehmung (92) aufweist, wobei das Verbindungselement (90) für eine Anordnung des Griffelements (10) an dem Adapterelement (24) ausgebildet ist, und
wobei die Wirkverbindung mit dem zumindest einen Sensormittel (110, 112, 118, 120, 122, 124, 126, 128) durch ein elastisches Material realisiert wird.

2. Griffelement nach Anspruch 1, wobei das Griffelement im Wesentlichen aus dem elastischen Material hergestellt ist.

3. Griffelement nach einem der Ansprüche 1 bis 2, wobei die zumindest eine Aufnahme (68, 130, 144, 164, 184, 200) so ausgestaltet ist, dass eine Bewegung von zumindest einem Finger (106, 108) des Benutzers in der zumindest einen Aufnahme (68, 130, 144, 164, 184, 200) über das Verbindungselement (90, 146, 168, 206) durch das zumindest eine Sensormittel (110, 112, 118, 120, 122, 124, 126, 128), das an dem Adapterelement (24, 26, 28, 30, 32) angeordnet ist, detektierbar ist.

4. Griffelement nach einem der Ansprüche 1 bis 3, wobei das Griffelement das zumindest eine Sensormittel (110, 112, 118, 120, 122, 124, 126, 128) zur Detektion von zumindest der zumindest einen Bewegung von dem zumindest einen Finger (106, 108) des Benutzers in der Aufnahme (68, 130, 144, 164, 184, 200) aufweist.

5. Griffelement nach Anspruch 4, wobei das zumindest eine Sensormittel (110, 112, 118, 120, 122, 124, 126, 128) zumindest einen Sensor (112, 118, 120, 122, 124, 126, 128), vorzugsweise zumindest einen Drucksensor, einen Kraftsensor oder einen Bewegungssensor aufweist.

6. Griffelement nach einem der Ansprüche 1 bis 5, wobei die zumindest eine Aufnahme (68, 130, 144, 164, 184, 200) ferner so ausgestaltet ist, dass sie eine Übermittlung zumindest einer Force-Feedback-Information zu dem zumindest einen Finger (106, 108) des Benutzers, der in der zumindest einen Aufnahme (68, 130, 144, 164, 184, 200) aufgenommen ist, ermöglicht.

7. Griffelement nach Anspruch 6, wobei die zumindest eine Aufnahme (68, 130, 144, 164, 184, 200) so ausgestaltet ist, dass die zumindest eine Force-Feedback-Information von dem Adapterelement (24, 26, 28, 30, 32) über das Verbindungselement (90, 146, 168, 206) an den zumindest einen Finger (106, 108) des Benutzers, der in der zumindest einen Aufnahme (68, 130, 144, 164, 184, 200) aufgenommen ist, weitergebbar ist.

8. Griffelement nach einem der Ansprüche 6 bis 7, wobei das Griffelement zumindest einen Aktuator (150, 152, 154, 186, 208) aufweist, über die zumindest eine Force-Feedback-Information an den zumindest einen Finger (106, 108) des Benutzers, der in der zumindest einen Aufnahme (68, 130, 144, 164, 184, 200) aufgenommen ist, weitergebbar ist.

9. Griffelement nach einem der Ansprüche 1 bis 8, wobei das Griffelement zumindest ein passives Force-Feedback-Element aufweist.

10. Greifereingabemodul mit einem Griffelement (10, 12, 14, 16, 18, 20) nach einem der Ansprüche 1 bis 9 und einem Adapterelement (24, 26, 28, 30, 32) zur Aufnahme des Griffelements (10, 12, 14, 16, 18, 20) zur Bedienung eines haptischen Eingabesystems (54) zur Steuerung von zumindest einem Objekt (220), wobei das Adapterelement (24, 26, 28, 30, 32) eine Schnittstelle (104) zur Kommunikation mit dem haptischen Eingabesystem (54) oder dem zumindest einen Objekt (220) und zumindest ein Aufnahmeelement (96, 116, 132, 174, 204) zur Aufnahme des Griffelements (10, 12, 14, 16, 18, 20) an dem Adapterelement (24, 26, 28, 30, 32) aufweist und wobei entweder das Griffelement (10, 12, 14, 16, 18, 20) und/oder das Adapterelement (24, 26, 28, 30, 32) mit zumindest einem Sensormittel (110, 112, 118, 120, 122, 124, 126, 128) in Wirkverbindung steht.

11. Haptisches Eingabesystem zur Steuerung von zumindest einem Objekt (220), mit einem Greifereingabemodul (40, 42, 44, 46, 48) nach Anspruch 10.

12. Medizinisches Instrumentensystem, mit
- zumindest einem medizinischen Instrument (220), und
- zumindest einem haptischen Eingabesystem (54) nach Anspruch 11,
wobei das zumindest eine medizinische Instrument (220) durch das haptische Eingabesystem (54) steuerbar ist (222).

## Claims

1. Handle element for arrangement on an adapter element to form a gripper input module for a haptic input system for controlling at least one object, the handle element having at least one connecting element (90, 146, 168, 206) for arranging the handle element on the adapter element (24, 26, 28, 30, 32),
comprising at least one receptacle (68, 130, 144, 164, 184, 200) for receiving at least two fingers (106, 108) of a user therein, said receptacle being operatively connected at least in one portion to at least one sensor means (110, 112, 118, 120, 122, 124, 126, 128),
wherein the at least one receptacle (68, 130, 144, 164, 184, 200) is configured such that movement information of a movement of at least one finger (106, 108) of the user in the receptacle (68, 130, 144, 164, 184, 200) can be detected by the sensor means (110, 112, 118, 120, 122, 124, 126, 128) and thus the movement information can be transmitted for controlling the at least one object,
wherein the handle element (10) comprises a spherical body (88), wherein a spherical surface of the body (88) has at least one opening for the at least one receptacle (68, 130, 144, 164, 184, 200)
wherein the spherical body (88) has the connecting element (90) in the form of a recess (92), the connecting element (90) being configured for arrangement of the handle element (10) on the adapter element (24), and
wherein the operative connection with the at least one sensor means (110, 112, 118, 120, 122, 124, 126, 128) is realized by an elastic material.

2. Handle element according to claim 1, wherein the handle element is substantially made of the elastic material.

3. Handle element according to any one of claims 1 to 2, wherein the at least one receptacle (68, 130, 144, 164, 184, 200) is configured such that movement of at least one finger (106, 108) of the user in the at least one receptacle (68, 130, 144, 164, 184, 200) can be detected via the connecting element (90, 146, 168, 206) by the at least one sensor means (110, 112, 118, 120, 122, 124, 126, 128) arranged on the adapter element (24, 26, 28, 30, 32).

4. Handle element according to any one of claims 1 to 3, wherein said handle element comprises said at least one sensor means (110, 112, 118, 120, 122, 124, 126, 128) for detecting the at least one movement of the at least one finger (106, 108) of the user in the receptacle (68, 130, 144, 164, 184, 200).

5. Handle element according to claim 4, wherein the at least one sensor means (110, 112, 118, 120, 122, 124, 126, 128) comprises at least one sensor (112, 118, 120, 122, 124, 126, 128), preferably at least one pressure sensor, force sensor or motion sensor.

6. Handle element according to any one of claims 1 to 5, wherein the at least one receptacle (68, 130, 144, 164, 184, 200) is further configured to allow transmission of at least one force feedback information to the at least one finger (106, 108) of the user received in the at least one receptacle (68, 130, 144, 164, 184, 200).

7. Handle element according to claim 6, wherein the at least one receptacle (68, 130, 144, 164, 184, 200) is configured such that the at least one force feedback information is transmitted from the adapter element (24, 26, 28, 30, 32) via the connecting element (90, 146, 168, 206) to the at least one finger (106, 108) of the user accommodated in the at least one receptacle (68, 130, 144, 164, 184, 200).

8. Handle element according to one of claims 6 to 7, wherein the handle element comprises at least one actuator (150, 152, 154, 186, 208) via which at least one force feedback information can be passed on to the at least one finger (106, 108) of the user which is accommodated in the at least one receptacle (68, 130, 144, 164, 184, 200).

9. Handle element according to any one of claims 1 to 8, wherein the handle element comprises at least one passive force feedback element.

10. Gripper input module with a handle element (10, 12, 14, 16, 18, 20) according to one of claims 1 to 9 and an adapter element (24, 26, 28, 30, 32) for receiving the handle element (10, 12, 14, 16, 18, 20) for operating a haptic input system (54) for controlling at least one object (220), wherein the adapter element (24, 26, 28, 30, 32) comprises an interface (104) for communication with the haptic input system (54) or the at least one object (220) and at least one receiving element (96, 116, 132, 174, 204) for receiving the handle element (10, 12, 14, 16, 18, 20) on the adapter element (24, 26, 28, 30, 32) and wherein either the handle element (10, 12, 14, 16, 18, 20) and/or the adapter element (24, 26, 28, 30, 32) is operatively connected to at least one sensor means (110, 112, 118, 120, 122, 124, 126, 128).

11. Haptic input system for controlling at least one object (220), comprising a gripper input module (40, 42, 44, 46, 48) according to claim 10.

12. Medical instrument system, with
- at least one medical instrument (220), and
- at least one haptic input system (54) according to claim 11,
wherein the at least one medical instrument (220) is controllable (222) by the haptic input system (54).

## Revendications

1. Élément formant poignée destiné à être monté sur un élément adaptateur en vue de former un module d'entrée à préhension pour un système d'entrée haptique servant à commander au moins un objet, l'élément formant poignée avec au moins un élément de liaison (90, 146, 168, 206) destiné à monter l'élément formant poignée sur l'élément adaptateur (24, 26, 28, 30, 32),
comprenant au moins un logement (68, 130, 144, 164, 184, 200) destiné à accueillir au moins deux doigts (106, 108) d'un utilisateur dans celui-ci, le logement se trouvant, au moins dans une portion, en liaison fonctionnelle avec au moins un moyen de détection (110, 112, 118, 120, 122, 124, 126, 128),
l'au moins un logement (68, 130, 144, 164, 184, 200) étant configuré de telle sorte qu'une information de mouvement d'un mouvement d'au moins un doigt (106, 108) de l'utilisateur dans le logement (68, 130, 144, 164, 184, 200) peut être détectée par le moyen de détection (110, 112, 118, 120, 122, 124, 126, 128) et l'information de mouvement peut ainsi être transmise en vue de commander l'au moins un objet,
l'élément formant poignée (10) possédant un corps (88) sphérique, une surface sphérique du corps (88) possédant au moins une ouverture pour l'au moins un logement (68, 130, 144, 164, 184, 200),
le corps (88) sphérique possédant ledit élément de liaison (90) sous la forme d'un évidement (92), l'élément de liaison (90) étant configuré pour un montage de l'élément formant poignée (10) sur l'élément adaptateur (24),
la liaison fonctionnelle avec l'au moins un moyen de détection (110, 112, 118, 120, 122, 124, 126, 128) étant réalisée par un matériau élastique.

2. Élément formant poignée selon la revendication 1, l'élément formant poignée étant sensiblement fabriqué à partir du matériau élastique.

3. Élément formant poignée selon l'une des revendications 1 et 2, l'au moins un logement (68, 130, 144, 164, 184, 200) étant configuré de telle sorte qu'un mouvement d'au moins un doigt (106, 108) de l'utilisateur dans l'au moins un logement (68, 130, 144, 164, 184, 200) peut être détecté par le biais de l'élément de liaison (90, 146, 168, 206) par l'au moins un moyen de détection (110, 112, 118, 120, 122, 124, 126, 128) qui est monté sur l'élément adaptateur (24, 26, 28, 30, 32).

4. Élément formant poignée selon l'une des revendications 1 à 3, l'élément formant poignée possédant l'au moins un moyen de détection (110, 112, 118, 120, 122, 124, 126, 128) destiné à la détection de l'au moins un mouvement de l'au moins un doigt (106, 108) de l'utilisateur dans le logement (68, 130, 144, 164, 184, 200).

5. Élément formant poignée selon la revendication 4, l'au moins un moyen de détection (110, 112, 118, 120, 122, 124, 126, 128) possédant au moins un capteur (112, 118, 120, 122, 124, 126, 128), de préférence au moins un capteur de pression, un capteur de force ou un capteur de mouvement.

6. Élément formant poignée selon l'une des revendications 1 à 5, l'au moins un logement (68, 130, 144, 164, 184, 200) étant en outre configuré de telle sorte qu'il rend possible une communication d'au moins une information de rétroaction de force vers l'au moins un doigt (106, 108) de l'utilisateur qui est accueilli dans l'au moins un logement (68, 130, 144, 164, 184, 200).

7. Élément formant poignée selon la revendication 6, l'au moins un logement (68, 130, 144, 164, 184, 200) étant configuré de telle sorte que l'au moins une information de rétroaction de force peut être retransmise par le biais de l'élément de liaison (90, 146, 168, 206) de l'élément adaptateur (24, 26, 28, 30, 32) à l'au moins un doigt (106, 108) de l'utilisateur qui est accueilli dans l'au moins un logement (68, 130, 144, 164, 184, 200).

8. Élément formant poignée selon l'une des revendications 6 et 7, l'élément formant poignée possédant au moins un actionneur (150, 152, 154, 186, 208) par le biais duquel l'au moins une information de rétroaction de force peut être retransmise à l'au moins un doigt (106, 108) de l'utilisateur qui est accueilli dans l'au moins un logement (68, 130, 144, 164, 184, 200).

9. Élément formant poignée selon l'une des revendications 1 à 8, l'élément formant poignée possédant au moins un élément de rétroaction de force passif.

10. Module d'entrée à préhension comprenant un élément formant poignée (10, 12, 14, 16, 18, 20) selon l'une des revendications 1 à 9 et un élément adaptateur (24, 26, 28, 30, 32) destiné à accueillir l'élément formant poignée (10, 12, 14, 16, 18, 20) en vue de manipuler un système d'entrée haptique (54) servant à commander au moins un objet (220), l'élément adaptateur (24, 26, 28, 30, 32) possédant une interface (104) servant à la communication avec le système d'entrée haptique (54) ou l'au moins un objet (220) et au moins un élément d'accueil (96, 116, 132, 174, 204) destiné à accueillir l'élément formant poignée (10, 12, 14, 16, 18, 20) sur l'élément adaptateur (24, 26, 28, 30, 32) et l'élément formant poignée (10, 12, 14, 16, 18, 20) et/ou l'élément adaptateur (24, 26, 28, 30, 32) se trouvant en liaison fonctionnelle avec au moins un moyen de détection (110, 112, 118, 120, 122, 124, 126, 128).

11. Système d'entrée haptique destiné à commander au moins un objet (220), comprenant un module d'entrée à préhension (40, 42, 44, 46, 48) selon la revendication 10.

12. Système d'instrument médical, comprenant
- au moins un instrument médical (220) et
- au moins un système d'entrée haptique (54) selon la revendication 11, l'au moins un instrument médical (220) pouvant être commandé (222) par le système d'entrée haptique (54).
